# EUROPEAN PATENT SPECIFICATION

(11) **EP 1 490 105 B1**
(45) Date of publication and mention of the grant of the patent: **02.10.2013**
(21) Application number: 03744203.5
(22) Date of filing: 06.03.2003
(51) Int. Cl.: A61K 39/255, A01N 65/00, C12P 19/34, C12N 7/00, C12N 15/00, C12N 15/66, C07H 21/04

(54) **MULTIPLE AND MULTIVALENT DNA VACCINES IN OVO**
MULTIPLE UND MULTIVALENTE DNA-VAKZINE IN OVO
VACCINS A BASE D'ADN MULTIPLES ET MULTIVALENTS IN OVO

(30) Priority: 08.03.2002 US 362547 P; 04.03.2003 US 377718
(43) Date of publication of application: 29.12.2004
(73) Proprietor: Schweitzer Chemical Corporation USA, City of Industry, CA 91748 (US)
(72) Inventor: KUO, Tsun-Yung, 5F, No. 92, I-Lan 260 (TW)
(74) Representative: Viering, Jentschura & Partner
(86) International application number: PCT/US2003/006811
(87) International publication number: WO 2003/075843

(56) References cited:
- WO-A-98/56413
- US-A- 5 693 530
- US-A- 6 001 369
- KARACA K ET AL: "Recombinant fowlpox viruses coexpressing chicken type I IFN and Newcastle disease virus HN and F genes: influence of IFN on protective efficacy and humoral responses of chickens following in ovo or post-hatch administration of recombinant viruses" VACCINE, BUTTERWORTH SCIENTIFIC. GUILDFORD, GB, vol. 16, no. 16, October 1998 (1998-10), pages 1496-1503, XP004129200 ISSN: 0264-410X
- MCCMILLEN J K ET AL: "The safe and effective use of fowlpox virus as a vector for poultry vaccines" DEVELOPMENTS IN BIOLOGICAL STANDARDIZATION, KARGER, BASEL, CH, vol. 82, 23 May 1993 (1993-05-23), pages 137-145, XP002090394 ISSN: 0301-5149
- GAGIC M. ET AL.: 'In ovo vaccination of specific-pathogen-free chickens with vaccines containing multiple agents' AVIAN DISEASES vol. 43, 1999, pages 293 - 301, XP000982144
- SHARMA J.M.: 'Embryo vaccination with infectious bursal disease virus alone or in combination with Marek's disease vaccine' AVIAN DISEASES vol. 29, no. 4, 1985, pages 1155 - 1169, XP002074230
- SAHRMA J.M.: 'Embryo vaccination of specific-pathogen-free chickens with infectious bursal disease virus: tissue distribution of the vaccine virus and protection of hatched chickens against disease' AVIAN DISEASES vol. 30, no. 4, 1986, pages 776 - 780, XP002074232
- REDDY S.K. ET AL.: 'Protective efficacy of a recombinant herpesvirus of turkeys as an in ovo vaccine against Newcastle and Marek's diseases in specific-pathogen-free chickens' VACCINE vol. 14, no. 6, 1996, pages 469 - 477, XP004057273

### SEQUENCE LISTING

<110> KUO, Tsun Yuang
<120> MULTIPLE AND MULTIVALENT DNA VACCINES IN OVO
<130> 39734-186920
<160> 19
<170> PatentIn version 3.2
<210> 1
   <211> 3650
   <212> DNA
   <213> Marek's disease virus
<400> 1
   tcgagctcgc cggggatgtt tagtcacgat agacatcggt tcgcccagcc gtcgaataca    60
   gcattatatt ttagtgttga aaatgtaggg ctgcttcctc acttaaagga ggaaatggct   120
   cgattcatgt ttcatagcag tagaaaaaca gattggaccg tcagtaagtt tagagggttt   180
   tatgacttta gcactataga taatgtaact gcggcccatc gcatggcttg gaaatatatc   240
   aaagaactga tttttgcaac agctttattt tcttctgtat ttaaatgtgg cgaattgcac   300
   atctgtcgtg ccgacagttt gcagatcaac agcaatggag actatgtatg gaaaaatgga   360
   atatatataa catatgaaac cgaatatcca cttataatga ttctggggtc agaatcaagc   420
   acttcagaaa cgcaaaatat gactgcaatt attgatacag atgttttttc gttgctttat   480
   tctattttgc agtatatggc ccccgttacg gcagatcagg tgcgagtaga acagattacc   540
   aacagccacg cccccatctg acccgtccaa tattcttgtg tccctgcatt ttatctcaca   600
   caatttatga acagcatcat taagatcatc tcactatgca ctattttagg cggaattgca   660
   tttttttcct tatagttatt ctatatggta cgaactcatc tccgagtacc caaaatgtga   720
   catcaagaga agttgtttcg agcgtccagt tgtctgagga agagtctacg ttttatcttt   780
   gtcccccacc agtgggttca accgtgatcc gtctagaacc gccgcgaaaa tgtcccgaac   840
   ctagaaaagc caccgagtgg ggtgaaggaa tcgcgatatt atttaaagag aatatcagtc   900
   catataaatt taaagtgacg ctttattata aaaatatcat tcagacgacg acatggacgg   960
   ggacgacata tagacagatc actaatcgat atacagatag gacgcccgtt tccattgaag  1020
   agatcacgga tctaatcgac ggcaaaggaa gatgctcatc taaagcaaga taccttagaa  1080
   acaatgtata tgttgaagcg tttgacaggg atgcgggaga aaaacaagta cttctaaaac  1140
   catcaaaatt caacacgccc gaatctaggg catggcacac gactaatgag acgtataccg  1200
   tgtggggatc accatggata tatcgaacgg gaacctccgt caattgtata gtagaggaaa  1260
   tggatgcccg ctctgtgttt ccgtattcat attttgcaat ggccaatggc gacatcgcga 1320
   acatatctcc attttatggt ctatccccac cagaggctgc cgcagaaccc atgggatatc 1380
   cccaggataa tttcaaacaa ctagatagct atttttcaat ggatttggac aagcgtcgaa 1440
   aagcaagcct tccagtcaag cgtaactttc tcatcacatc acacttcaca gttgggtggg 1500
   actgggctcc aaaaactact cgtgtatgtt caatgactaa gtggaaagag gtgactgaaa 1560
   tgttgcgtgc aacagttaat gggagataca gatttatggc ccgtgaactt tcggcaacgt 1620
   ttatcagtaa tacgactgag tttgatccaa atcgcatcat attaggacaa tgtattaaac 1680
   gcgaggcaga agcagcaatc gagcagatat ttaggacaaa atataatgac agtcacgtca 1740
   aggttggaca tgtacaatat ttcttggctc tcgggggatt tattgtagca tatcagcctg 1800
   ttctatccaa atccctggct catatgtacc tcagagaatt gatgagagac aacaggaccg 1860
   atgagatgct cgacctggta aacaataagc atgcaattta taagaaaaat gctacctcat 1920
   tgtcacgatt gcggcgagat attcgaaatg caccaaatag aaaaataaca ttagacgaca 1980
   ccacagctat taaatcgaca tcgtctgttc aattcgccat gctccaattt ctttatgatc 2040
   atatacaaac ccatattaat gatatgttta gtaggattgc cacagcttgg tgcgaattgc 2100
   agaatagaga acttgtttta tggcacgaag ggataaagat taatcctagc gctacagcga 2160
   gtgcaacatt aggaaggaga gtggctgcaa agatgttggg ggatgtcgct gctgtatcga 2220
   gctgcactgc tatagatgcg gaatccgtca ctttgcaaaa ttctatgcga gttatcacat 2280
   ccactaatac atgttatagc cgaccattgg ttctattttc atatggagaa aaccaaggaa 2340
   acatacaggg acaactcggt gaaaacaacg agttgcttcc aacgctagag gctgtagagc 2400
   catgctcggc taatcatcgt agatattttc tgtttggatc cggttatgct ttatttgaaa 2460
   actataattt tgttaagatg gtagacgctg ccgatataca gattgctagc acatttgtcg 2520
   agcttaatct aaccctgcta gaagatcggg aaattttgcc tttatccgtt tacacaaaag 2580
   aagagttgcg tgatgttggt gtattggatt atgcagaagt agctcgccgc aatcaactac 2640
   atgaacttaa attttatgac ataaacaaag taatagaagt ggatacaaat tacgcgttta 2700
   tgaacggttt ggccgaattg tttaacggta tgggtcaggt agggcaagct ataggcaaag 2760
   ttgtagtagg ggctgccggt gcaatcgtat ctaccatatc tggtgtctct gctttcatgt 2820
   caatcccttt ggggctttcg gcaatcggtt taatcattat agcaggactc gtggctgcat 2880
   ttttagcata tcgttatgta aacaagctta aaagcaatcc aatgaaagcc ctttatccta 2940
   tgacaacaga agtgcttaag gcacaggcaa cgcgtgagtt gcatggcgag gaatcagatg 3000
   atttggaacg aacatctatt gatgaaagaa aattagaaga agctagagaa atgataaaat 3060
   atatggcgtt agtctccgcg gaagaacgcc acgagaaaaa actgcggaga aagaggcgag   3120
   gcactaccgc cgttctatcg gaccacctgg caaaaatgag gattaaaaat agtaacccta   3180
   aatatgataa gttacctact acatattcag actcagaaga tgatgctgtg taagtgggca   3240
   ctattatatt tgaactgaat aaaacgcata gagcatgata tggtttactc atttattgcg   3300
   agatataaag catattcaat acgatatatt gcgaacgtga tgctaaaaac atagctccct   3360
   gtattattga tgcgccatca tttgattaat aaatacatcg acgccggcat cactggtgcg   3420
   gtgtatacca gctacggcgc tagcattcat ggtatcccgt gattgctcga tgctttcctt   3480
   ctgaattccg tcggaacgct cctgagagat ggtcgcagtt attggtacat ttcgaccagc   3540
   ctccggatct gaaactggca caggaatgca ccgtggaatt ggtagaagtt tttccttccg   3600
   tggaaggcat agggcgttcg actcccatgg gccatgaaac tgtgggatgt              3650
<210> 2
   <211> 3004
   <212> DNA
   <213> infectious bursal disease virus (IBDV)
<400> 2
   tgatgccaac aaccggaccg gcgtccattc cggacgacac cctggagaag cacactctca     60
   ggtcagagac ctcgacctac aatttgactg tgggggacac agggtcaggg ctaattgtct    120
   ttttccctgg attccctggc tcaattgtgg gtgctcacta cacactgcag agcaatggga    180
   actacaagtt cgatcagatg ctcctgactg cccagaacct accggccagt tacaactact    240
   gcaggctagt gagtcggagt ctcacagtga ggtcaagcac acttcctggt ggcgtttatg    300
   cactaaacgg caccataaac gccgtgacct tccaaggaag cctgagtgaa ctgacagatg    360
   ttagctacaa tgggttgatg tctgcaacag ccaacatcaa cgacaaaatt gggaacgtcc    420
   tagtagggga aggggtcacc gtcctcagct tacccacatc atatgatctt gggtatgtga    480
   ggcttggtga ccccattccc gcaatagggc ttgacccaaa aatggtagcc acatgtgaca    540
   gcagtgacag gcccagagtc tacaccataa ctgcagccga tgattaccaa ttctcatcac    600
   agtaccaacc aggtggggta acaatcacac tgttctcagc caacattgat gccatcacaa    660
   gcctcagcgt tgggggagag ctcgtgtttc gaacaagcgt ccacggcctt gtactgggcg    720
   ccaccatcta cctcataggc tttgatggga caacggtaat caccagggct gtggccgcaa    780
   acactgggct gacgaccggc accgacaacc ttatgccatt caatcttgtg attccaacaa    840
   acgagataac ccagccaatc acatccatca aactggagat agtgacctcc aaaagtggtg    900
   gtcaggcagg ggatcagatg ttatggtcgg caagagggag cctagcagtg acgatccatg    960
   gtggcaacta tccaggggcc ctccgtcccg tcacgctagt ggcctacgaa agagtggcaa   1020
   caggatccgt cgttacggtc gctggggtga gcaacttcga gctgatccca aatcctgaac 1080
   tagcaaagaa cctggttaca gaatacggcc gatttgaccc aggagccatg aactacacaa 1140
   aattgatact gagtgagagg gaccgtcttg gcatcaagac cgtctggcca acaagggagt 1200
   acactgactt tcgtgaatac ttcatggagg tggccgacct caactctccc ctgaagattg 1260
   caggagcatt cggcttcaaa gacataatcc gggccataag gaggatagct gtgccggtgg 1320
   tctccacatt gttcccacct gccgctcccc tagcccatgc aattggggaa ggtgtagact 1380
   acctgctggg cgatgaggca caggctgctt caggaactgc tcgagccgcg tcaggaaaag 1440
   caagagctgc ctcaggccgc ataaggcagc tgactctcgc cgccgacaag ggtacgaggg 1500
   tagtcgcgaa tctattccag gtgccccaga atcccgtagt cgacgggatt cttgcttcac 1560
   ctggggtact ccgcggtgca cacaacctcg actgcgtgtt aagagagggt gccacgctat 1620
   tccctgtggt tattacgaca gtggaagacg ccatgacacc caaagcattg aacagcaaaa 1680
   tgtttgctgt cattgaaggc gtgcgagaag acctccaacc tccatctcaa agaggatcct 1740
   tcatacgaac tctctctgga cacagagtct atggatatgc tccagatggg gtacttccac 1800
   tggagactgg gagagactac accgttgtcc caatagatga tgtctgggac gacagcatta 1860
   tgctgtccaa agatcccata cctcctattg tgggaaacag tggaaatcta gccatagctt 1920
   acatggatgt gtttcgaccc aaagtcccaa tccatgtggc tatgacggga gccctcaatg 1980
   cttgtggcga gattgagaaa gtaagcttta gaagcaccaa gctcgccact gcacaccgac 2040
   ttggccttaa gttggctggt cccggagcat tcgatgtaaa caccgggccc aactgggcaa 2100
   cgttcatcaa acgtttccct cacaatccac gcgactggga caggctcccc tacctcaacc 2160
   taccatacct tccacccaat gcaggacgcc agtaccacct tgccatggct gcatcagagt 2220
   tcaaagagac ccccgaactc gagagtgccg tcagagcaat ggaagcagca gccaacgtgg 2280
   acccactatt ccaatctgca ctcagtgtgt tcatgtggct ggaagagaat gggattgtga 2340
   ctgacatggc caacttcgca ctcagcgacc cgaacgccca tcggatgcga aattttcttg 2400
   caaacgcacc acaagcaggc agcaagtcgc aaagggccaa gtacgggaca gcaggctacg 2460
   gagtggaggc tcggggcccc acaccagagg aagcacagag ggaaaaagac acacggatct 2520
   caaagaagat ggagaccatg ggcatctact ttgcaacacc agaatgggta gcactcaatg 2580
   ggcaccgagg gccaagcccc ggccagctaa agtactggca gaacacacga gaaataccgg 2640
   acccaaacga ggactatcta gactacgtgc atgcagagaa gagccggttg gcatcagaag 2700
   aacaaatcct aagggcagct acgtcgatct acggggctcc aggacaggca gagccacccc 2760
   aagctttcat agacgaagtt gccaaagtct atgaaatcaa ccatggacgt ggcccaaacc 2820
   aagaacagat gaaagatctg ctcttgactg cgatggagat gaagcatcgc aatcccaggc 2880
   gggctctacc aaagcccaag ccaaaaccca atgctccaac acagagaccc cctggtcggc 2940
   tgggccgctg gatcaggacc gtctctgatg aggaccttga gtgaggctcc tggaagtctc 3000
   ccga 3004
<210> 3
   <211> 15186
   <212> DNA
   <213> Newcastle disease virus (NDV)
<400> 3
   accaaacaga gaatccgtga gttacgataa aaggcgaagg agcaattgaa gtcgcacggg 60
   tagaaggtgt gaatctcgag tgcgagcccg aagcacaaac tcgagaaagc cttctgccaa 120
   catgtcttcc gtatttgatg agtacgaaca gctcctcgcg gctcagactc gccccaatgg 180
   agctcatgga gggggagaaa aagggagtac cttaaaagta gacgtcccgg tattcactct 240
   taacagtgat gacccagaag atagatggag ctttgtggta ttctgcctcc ggattgctgt 300
   tagcgaagat gccaacaaac cactcaggca aggtgctctc atatctcttt tatgctccca 360
   ctcacaggta atgaggaacc atgttgccat tgcagggaaa cagaatgaag ccacattggc 420
   cgtgcttgag attgatggct ttgccaacgg cacgccccag ttcaacaata ggagtggagt 480
   gtctgaagag agagcacaga gatttgcgat gatagcagga tctctccctc gggcatgcag 540
   caacggaacc ccgttcgtca cagccggggc agaagatgat gcaccagaag acatcaccga 600
   taccctggag aggatcctct ctatccaggc tcaagtatgg gtcacagtag caaaagccat 660
   gactgcgtat gagactgcag atgagtcgga aacaaggcga atcaataagt atatgcagca 720
   aggcagggtc caaaagaaat acatcctcta ccccgtatgc aggagcacaa tccaactcac 780
   gatcagacag tctcttgcag tccgcatctt tttggttagc gagctcaaga gaggccgcaa 840
   cacggcaggt ggtacctcta cttattataa cctggtaggg gacgtagact catacatcag 900
   gaataccggg cttactgcat tcttcttgac actcaagtac ggaatcaaca ccaagacatc 960
   agcccttgca cttagtagcc tctcaggcga catccagaag atgaagcagc tcatgcgttt 1020
   gtatcggatg aaaggagata atgcgccgta catgacatta cttggtgata gtgaccagat 1080
   gagctttgcg cctgccgagt atgcacaact ttactccttt gccatgggta tggcatcagt 1140
   cctagataaa ggtactggga aataccaatt tgccagggac tttatgagca catcattctg 1200
   gagacttgga gtagagtacg ctcaggctca gggaagtagc attaacgagg atatggctgc 1260
   cgagctaaag ctaaccccag cagcaatgaa gggcctggca gctgctgccc aacgggtctc 1320
   cgacgatacc agcagcatat acatgcctac tcaacaagtc ggagtcctca ctgggcttag 1380
   cgaggggggg tcccaagctc tacaaggcgg atcgaataga tcgcaagggc aaccagaagc 1440
   cggggatggg gagacccaat tcctggatct gatgagagcg gtagcaaata gcatgaggga 1500
   ggcgccaaac tctgcacagg gcactcccca atcggggcct cccccaactc ctgggccatc 1560
   ccaagataac gacaccgact gggggtattg atggacaaaa cccagcctgc ttccacaaaa 1620
   acatcccaat gccctcaccc gtagtcgacc cctcgatttg cggctctata tgaccacacc 1680
   ctcaaacaaa catccccctc tttcctccct ccccctgctg tacaactccg cacgccctag 1740
   ataccacagg cacaatgcgg ctcactaaca atcaaaacag agccgaggga attagaaaaa 1800
   agtacgggta gaagagggat attcagagat cagggcaagt ctcccgagtc tctgctctct 1860
   cctctacctg atagaccagg acaaacatgg ccacctttac agatgcagag atcgacgagc 1920
   tatttgagac aagtggaact gtcattgaca acataattac agcccagggt aaaccagcag 1980
   agactgttgg aaggagtgca atcccacaag gcaagaccaa ggtgctgagc gcagcatggg 2040
   agaagcatgg gagcatccag ccaccggcca gtcaagacaa ccccgatcga caggacagat 2100
   ctgacaaaca accatccaca cccgagcaaa cgaccccgca tgacagcccg ccggccacat 2160
   ccgccgacca gccccccacc caggccacag acgaagccgt cgacacacag ttcaggaccg 2220
   gagcaagcaa ctctctgctg ttgatgcttg acaagctcag caataaatcg tccaatgcta 2280
   aaaagggccc atggtcgagc ccccaagagg ggaatcacca acgtccgact caacagcagg 2340
   ggagtcaacc cagtcgcgga aacagtcagg aaagaccgca gaaccaagtc aaggccgccc 2400
   ctggaaacca gggcacagac gtgaacacag catatcatgg acaatgggag gagtcacaac 2460
   tatcagctgg tgcaacccct catgctctcc gatcaaggca gagccaagac aatacccttg 2520
   tatctgcgga tcatgtccag ccacctgtag actttgtgca agcgatgatg tctatgatgg 2580
   aggcgatatc acagagagta agtaaggttg actatcagct agatcttgtc ttgaaacaga 2640
   catcctccat ccctatgatg cggtccgaaa tccaacagct gaaaacatct gttgcagtca 2700
   tggaagccaa cttgggaatg atgaagattc tggatcccgg ttgtgccaac atttcatctc 2760
   tgagtgatct acgggcagtt gcccgatctc acccggtttt agtttcaggc cctggagacc 2820
   cctctcccta tgtgacacaa ggaggcgaaa tggcacttaa taaactttcg caaccagtgc 2880
   cacatccatc tgaattgatt aaacccgcca ctgcatgcgg gcctgatata ggagtggaaa 2940
   aggacactgt ccgtgcattg atcatgtcac gcccaatgca cccgagttct tcagccaagc 3000
   tcctaagcaa gttagatgca gccgggtcga tcgaggaaat caggaaaatc aagcgccttg 3060
   ctctaaatgg ctaattacta ctgccacacg tagcgggtcc ctgtccactc ggcatcacac 3120
   ggaatctgca ccgagttccc ccccgcagac ccaaggtcca actctccaag cggcaatcct 3180
   ctctcgcttc ctcagcccca ctgaatggtc gcgtaaccgt aattaatcta gctacattta 3240
   agattaagaa aaaatacggg tagaattgga gtgccccaat tgtgccaaga tggactcatc 3300
   taggacaatt gggctgtact ttgattctgc ccattcttct agcaacctgt tagcatttcc 3360
   gatcgtccta caaggcacag gagatgggaa gaagcaaatc gccccgcaat ataggatcca 3420
   gcgccttgac ttgtggactg atagtaagga ggactcagta ttcatcacca cctatggatt 3480
   catctttcaa gttgggaatg aagaagccac tgtcggcatg atcgatgata aacccaagcg 3540
   cgagttactt tccgctgcga tgctctgcct aggaagcgtc ccaaataccg gagaccttat 3600
   tgagctggca agggcctgtc tcactatgat agtcacatgc aagaagagtg caactaatac 3660
   tgagagaatg gttttctcag tagtgcaggc accccaagtg ctgcaaagct gtagggttgt 3720
   ggcaaacaaa tactcatcag tgaatgcagt caagcacgtg aaagcgccag agaagattcc 3780
   cgggagtgga accctagaat acaaggtgaa ctttgtctcc ttgactgtgg taccgaagaa 3840
   ggatgtctac aagatcccag ctgcagtatt gaaggtttct ggctcgagtc tgtacaatct 3900
   tgcgctcaat gtcactatta atgtggaggt agacccgagg agtcctttgg ttaaatcttt 3960
   gtctaagtct gacagcggat actatgctaa cctcttcttg catattggac ttatgaccac 4020
   cgtagatagg aaggggaaga aagtgacatt tgacaagctg gaaaagaaaa taaggagcct 4080
   tgatctatct gtcgggctca gtgatgtgct cgggccttcc gtgttggtaa aagcaagagg 4140
   tgcacggact aagcttttgg cacctttctt ctctagcagt gggacagcct gctatcccat 4200
   agcaaatgct tctcctcagg tggccaagat actctggagt caaaccgcgt gcctgcggag 4260
   cgttaaaatc attatccaag caggtaccca acgcgctgtc gcagtgaccg ccgaccacga 4320
   ggttacctct actaagctgg agaaggggca cacccttgcc aaatacaatc cttttaagaa 4380
   ataagctgcg tctctgagat tgcgctccgc ccactcaccc agatcatcat gacacaaaaa 4440
   actaatctgt cttgattatt tacagttagt ttacctgtct atcaagttag aaaaaacacg 4500
   ggtagaagat tctggatccc ggttggcgcc ctccaggtgc aagatgggct ccagaccttc 4560
   taccaagaac ccagcaccta tgatgctgac tatccgggtt gcgctggtac tgagttgcat 4620
   ctgtccggca aactccattg atggcaggcc tcttgcagct gcaggaattg tggttacagg 4680
   agacaaagcc gtcaacatat acacctcatc ccagacagga tcaatcatag ttaagctcct 4740
   cccgaatctg cccaaggata aggaggcatg tgcgaaagcc cccttggatg catacaacag 4800
   gacattgacc actttgctca ccccccttgg tgactctatc cgtaggatac aagagtctgt 4860
   gactacatct ggagggggga gacaggggcg ccttataggc gccattattg gcggtgtggc 4920
   tcttggggtt gcaactgccg cacaaataac agcggccgca gctctgatac aagccaaaca 4980
   aaatgctgcc aacatcctcc gacttaaaga gagcattgcc gcaaccaatg aggctgtgca 5040
   tgaggtcact gacggattat cgcaactagc agtggcagtt gggaagatgc agcagtttgt 5100
   taatgaccaa tttaataaaa cagctcagga attagactgc atcaaaattg cacagcaagt 5160
   tggtgtagag ctcaacctgt acctaaccga attgactaca gtattcggac cacaaatcac 5220
   ttcacctgct ttaaacaagc tgactattca ggcactttac aatctagctg gtggaaatat 5280
   ggattactta ttgactaagt taggtgtagg gaacaatcaa ctcagctcat taatcggtag 5340
   cggcttaatc accggtaacc ctattctata cgactcacag actcaactct tgggtataca 5400
   ggtaactcta ccttcagtcg ggaacctaaa taatatgcgt gccacctact tggaaacctt 5460
   atccgtaagc acaaccaggg gatttgcctc ggcacttgtc cccaaagtgg tgacacaggt 5520
   cggttctgtg atagaagaac ttgacacctc atactgtata gaaactgact tagatttata 5580
   ttgtacaaga atagtaacgt tccctatgtc ccctggtatt tattcctgct tgagcggcaa 5640
   tacgtcggcc tgtatgtact caaagaccga aggcgcactt actacaccat acatgactat 5700
   caaaggttca gtcatcgcca actgcaagat gacaacatgt agatgtgtaa accccccggg 5760
   tatcatatcg caaaactatg gagaagccgt gtctctaata gataaacaat catgcaatgt 5820
   tttatcctta ggcgggataa ctttaaggct cagtggggaa ttcgatgtaa cttatcagaa 5880
   gaatatctca atacaagatt ctcaagtaat aataacaggc aatcttgata tctcaactga 5940
   gcttgggaat gtcaacaact cgatcagtaa tgctttgaat aagttagagg aaagcaacag 6000
   aaaactagac aaagtcaatg tcaaactgac tagcacatct gctctcatta cctatatcgt 6060
   tttgactatc atatctcttg tttttggtat acttagcctg attctagcat gctacctaat 6120
   gtacaagcaa aaggcgcaac aaaagacctt attatggctt gggaataata ctctagatca 6180
   gatgagagcc actacaaaaa tgtgaacaca gatgaggaac gaaggtttcc ctaatagtaa 6240
   tttgtgtgaa agttctggta gtctgtcagt tcagagagtt aagaaaaaac taccggttgt 6300
   agatgaccaa aggacgatat acgggtagaa cggtaagaga ggccgcccct caattgcgag 6360
   ccaggcttca caacctccgt tctaccgctt caccgacaac agtcctcaat catggaccgc 6420
   gccgttagcc aagttgcgtt agagaatgat gaaagagagg caaaaaatac atggcgcttg 6480
   atattccgga ttgcaatctt attcttaaca gtagtgacct tggctatatc tgtagcctcc 6540
   cttttatata gcatgggggc tagcacacct agcgatcttg taggcatacc gactaggatt 6600
   tccagggcag aagaaaagat tacatctaca cttggttcca atcaagatgt agtagatagg 6660
   atatataagc aagtggccct tgagtctccg ttggcattgt taaatactga gaccacaatt 6720
   atgaacgcaa taacatctct ctcttatcag attaatggag ctgcaaacaa cagtgggtgg 6780
   ggggcaccta tccatgaccc agattatata ggggggatag gcaaagaact cattgtagat 6840
   gatgctagtg atgtcacatc attctatccc tctgcatttc aagaacatct gaattttatc 6900
   ccggcgccta ctacaggatc aggttgcact cgaataccct catttgacat gagtgctacc 6960
   cattactgct acacccataa tgtaatattg tctggatgca gagatcactc acattcatat 7020
   cagtatttag cacttggtgt gctccggaca tctgcaacag ggagggtatt cttttctact 7080
   ctgcgttcca tcaacctgga cgacacccaa aatcggaagt cttgcagtgt gagtgcaact 7140
   cccctgggtt gtgatatgct gtgctcgaaa gtcacggaga cagaggaaga agattataac 7200
   tcagctgtcc ctacgcggat ggtacatggg aggttagggt tcgacggcca gtaccacgaa 7260
   aaggacctag atgtcacaac attattcggg gactgggtgg ccaactaccc aggagtaggg 7320
   ggtggatctt ttattgacag ccgcgtatgg ttctcagtct acggagggtt aaaacccaat 7380
   tcacccagtg acactgtaca ggaagggaaa tatgtgatat acaagcgata caatgacaca 7440
   tgcccagatg agcaagacta ccagattcga atggccaagt cttcgtataa gcctggacgg 7500
   tttggtggga aacgcataca gcaggctatc ttatctatca aggtgtcaac atccttaggc 7560
   gaagacccgg tactgactgt accgcccaac acagtcacac tcatgggggc cgaaggcaga 7620
   attctcacag tagggacatc tcatttcttg tatcaacgag ggtcatcata cttctctccc 7680
   gcgttattat atcctatgac agtcagcaac aaaacagcca ctcttcatag tccttataca 7740
   ttcaatgcct tcactcggcc aggtagtatc ccttgccagg cttcagcaag atgccccaac 7800
   tcgtgtgtta ctggagtcta tacagatcca tatcccctaa tcttctatag aaaccacacc 7860
   ttgcgagggg tattcgggac aatgcttgat ggtgtacaag caagacttaa ccctgcgtct 7920
   gcagtattcg atagcacatc ccgcagtcgc attactcgag tgagttcaag cagtaccaaa 7980
   gcagcataca caacatcaac ttgttttaaa gtggtcaaga ctaataagac ctattgtctc 8040
   agcattgctg aaatatctaa tactctcttc ggagaattca gaatcgtccc gttactagtt 8100
   gagatcctca aagatgacgg ggttagagaa gccaggtctg gctagttgag tcaattataa 8160
   aggagttgga aagatggcat tgtatcacct atcttctgcg acatcaagaa tcaaaccgaa 8220
   tgccggcgcg tgctcgaatt ccatgttgcc agttgaccac aatcagccag tgctcatgcg 8280
   atcagattaa gccttgtcat taatctcttg attaagaaaa aatgtaagtg gcaatgagat 8340
   acaaggcaaa acagctcatg gtaaataata cgggtaggac atggcgagct ccggtcctga 8400
   aagggcagag catcagatta tcctaccaga gccacacctg tcttcaccat tggtcaagca 8460
   caaactactc tattactgga aattaactgg gctaccgctt cctgatgaat gtgacttcga 8520
   ccacctcatt ctcagccgac aatggaaaaa aatacttgaa tcggcctctc ctgatactga 8580
   gagaatgata aaactcggaa gggcagtaca ccaaactctt aaccacaatt ccagaataac 8640
   cggagtgctc caccccaggt gtttagaaca actggctaat attgaggtcc cagattcaac 8700
   caacaaattt cggaagattg agaagaagat ccaaattcac aacacgagat atggagaact 8760
   gttcacaagg ctgtgtacgc atatagagaa gaaactgctg gggtcatctt ggtctaacaa 8820
   tgtcccccgg tcagaggagt tcagcagcat tcgtacggat ccggcattct ggtttcactc 8880
   aaaatggtcc acagccaagt ttgcatggct ccatataaaa cagatccaga ggcatctgat 8940
   ggtggcagct aagacaaggt ctgcggccaa caaattggtg atgctaaccc ataaggtagg 9000
   ccaagtcttt gtcactcctg aacttgtcgt tgtgacgcat acgaatgaga acaagttcac 9060
   atgtcttacc caggaacttg tattgatgta tgcagatatg atggagggca gagatatggt 9120
   caacataata tcaaccacgg cggtgcatct cagaagctta tcagagaaaa ttgatgacat 9180
   tttgcggtta atagacgctc tggcaaaaga cttgggtaat caagtctacg atgttgtatc 9240
   actaatggag ggatttgcat acggagctgt ccagctactc gagccgtcag gtacatttgc 9300
   aggagatttc ttcgcattca acctgcagga gcttaaagac attctaattg gcctcctccc 9360
   caatgatata gcagaatccg tgactcatgc aatcgctact gtattctctg gtttagaaca 9420
   gaatcaagca gctgagatgt tgtgtctgtt gcgtctgtgg ggtcacccac tgcttgagtc 9480
   ccgtattgca gcaaaggcag tcaggagcca aatgtgcgca ccgaaaatgg tagactttga 9540
   tatgatcctt caggtactgt ctttcttcaa gggaacaatc atcaacgggt acagaaagaa 9600
   gaatgcaggt gtgtggccgc gagtcaaagt ggatacaata tatgggaagg tcattgggca 9660
   actacatgca gattcagcag agatttcaca cgatatcatg ttgagagagt ataagagttt 9720
   atctgcactt gaatttgagc catgtataga atatgaccct gtcaccaacc tgagcatgtt 9780
   cctaaaagac aaggcaatcg cacaccccaa cgataattgg cttgcctcgt ttaggcggaa 9840
   ccttctctcc gaagaccaga agaaacatgt aaaagaagca acttcgacta atcgcctctt 9900
   gatagagttt ttagagtcaa atgattttga tccatataaa gagatggaat atctgacgac 9960
   ccttgagtac cttagagatg acaatgtggc agtatcatac tcgctcaagg agaaggaagt 10020
   gaaagttaat ggacggatct tcgctaagct gacaaagaag ttaaggaact gtcaggtgat 10080
   ggcggaaggg atcctagccg atcagattgc acctttcttt cagggaaatg gagtcattca 10140
   ggatagcata tccttgacca agagtatgct agcgatgagt caactgtctt ttaacagcaa 10200
   taagaaacgt atcactgact gtaaagaaag agtatcttca aaccgcaatc atgatccgaa 10260
   aagcaagaac cgtcggagag ttgcaacctt cataacaact gacctgcaaa agtactgtct 10320
   taattggaga tatcagacaa tcaaattgtt cgctcatgcc atcaatcagt tgatgggcct 10380
   acctcacttc ttcgaatgga ttcacctaag actgatggac actacgatgt tcgtaggaga 10440
   ccctttcaat cctccaagtg accctactga ctgtgacctc tcaagagtcc ctaatgatga 10500
   catatatatt gtcagtgcca gagggggtat cgaaggatta tgccagaagc tatggacaat 10560
   gatctcaatt gctgcaatcc aacttgctgc agctagatcg cattgtcgtg ttgcctgtat 10620
   ggtacagggt gataatcaag taatagcagt aacgagagag gtaagatcag acgactctcc 10680
   ggagatggtg ttgacacagt tgcatcaagc cagtgataat ttcttcaagg aattaattca 10740
   tgtcaatcat ttgattggcc ataatttgaa ggatcgtgaa accatcaggt cagacacatt 10800
   cttcatatac agcaaacgaa tcttcaaaga tggagcaatc ctcagtcaag tcctcaaaaa 10860
   ttcatctaaa ttagtgctag tgtcaggtga tctcagtgaa aacaccgtaa tgtcctgtgc 10920
   caacattgcc tctactgtag cacggctatg cgagaacggg cttcccaaag acttctgtta 10980
   ctatttaaac tatataatga gttgtgtgca gacatacttt gactctgagt tctccatcac 11040
   caacaattcg caccccgatc ttaatcagtc gtggattgag gacatctctt ttgtgcactc 11100
   atatgttctg actcctgccc aattaggggg actgagtaac cttcaatact caaggctcta 11160
   cactagaaat atcggtgacc cggggactac tgcttttgca gagatcaagc gactagaagc 11220
   agtgggatta ctgagtccta acattatgac taatatctta actaggccgc ctgggaatgg 11280
   agattgggcc agtctgtgca acgacccata ctctttcaat tttgagactg ttgcaagccc 11340
   aaatattgtt cttaagaaac atacgcaaag agtcctattt gaaacttgtt caaatccctt 11400
   attgtctgga gtgcacacag aggataatga ggcagaagag aaggcattgg ctgaattctt 11460
   gcttaatcaa gaggtgattc atccccgcgt tgcgcatgcc atcatggagg caagctctgt 11520
   aggtaggaga aagcaaattc aagggcttgt tgacacaaca aacaccgtaa ttaagattgc 11580
   gcttactagg aggccattag gcatcaagag gctgatgcgg atagtcaatt attctagcat 11640
   gcatgcaatg ctgtttagag acgatgtttt ttcctccagt agatccaacc accccttagt 11700
   ctcttctaat atgtgttctc tgacactggc agactatgca cggaatagaa gctggtcacc 11760
   tttgacggga ggcaggaaaa tactgggtgt atctaatcct gatacgatag aactcgtaga 11820
   gggtgagatt cttagtgtaa gcggagggtg tacaagatgt gacagcggag atgaacaatt 11880
   tacttggttc catcttccaa gcaatataga attgaccgat gacaccagca agaatcctcc 11940
   gatgagggta ccatatctcg ggtcaaagac acaggagagg agagctgcct cacttgcaaa 12000
   aatagctcat atgtcgccac atgtaaaggc tgccctaagg gcatcatccg tgttgatctg 12060
   ggcttatggg gataatgaag taaattggac tgctgctctt acgattgcaa aatctcggtg 12120
   taatgtaaac ttagagtatc ttcggttact gtccccttta cccacggctg ggaatcttca 12180
   acatagacta gatgatggta taactcagat gacattcacc cctgcatctc tctacaggtg 12240
   tcaccttaca ttcacatatc caatgattct caaaggctgt tcactgaaga aggagtcaaa 12300
   gaggggaatg tggtttacca acagagtcat gctcttgggt ttatctctaa tcgaatcgat 12360
   ctttccaatg acaacaacca ggacatatga tgagatcaca ctgcacctac atagtaaatt 12420
   tagttgctgt atcagagaag cacctgttgc ggttcctttc gagctacttg gggtggtacc 12480
   ggaactgagg acagtgacct caaataagtt tatgtatgat cctagccctg tatcggaggg 12540
   agactttgcg agacttgact tagctatctt caagagttat gagcttaatc tggagtcata 12600
   tcccacgata gagctaatga acattctttc aatatccagc gggaagttga ttggccagtc 12660
   tgtggtttct tatgatgaag atacctccat aaagaatgac gccataatag tgtatgacaa 12720
   tacccgaaat tggatcagtg aagctcagaa ttcagatgtg gtccgcctat ttgaatatgc 12780
   agcacttgaa gtgctcctcg actgttctta ccaactctat tacctgagag taagaggcct 12840
   agacaatatt gtcttatata tgggtgattt atacaagaat atgccaggaa ttctactttc 12900
   caacattgca gctacaatat ctcatcccgt cattcattca aggttacatg cagtgggcct 12960
   ggtcaaccat gacggatcac accaacttgc agatacggat tttatcgaaa tgtctgcaaa 13020
   actattagta tcttgcaccc gacgtgtgat ctccggctta tattcaggaa ataagtatga 13080
   tctgctgttc ccatctgtct tagatgataa cctgaatgag aagatgcttc agctgatatc 13140
   ccggttatgc tgtctgtaca cggtactctt tgctacaaca agagaaatcc cgaaaataag 13200
   aggcttaact gcagaagaga aatgttcaat actcactgag tatttactgt cggatgctgt 13260
   gaaaccatta cttagccccg atcaagtgag ctctatcatg tctcctaaca taattacatt 13320
   cccagctaat ctgtactaca tgtctcggaa gagcctcaat ttgatcaggg aaagggagga 13380
   cagggatact atcctggcgt tgttgttccc ccaagagcca ttattagagt tcccttctgt 13440
   gcaagatatt ggtgctcgag tgaaagatcc attcacccga caacctgcgg catttttgca 13500
   agagttagat ttgagtgctc cagcaaggta tgacgcattc acacttagtc agattcatcc 13560
   tgaactcaca tctccaaatc cggaggaaga ctacttagta cgatacttgt tcagagggat 13620
   agggactgca tcttcctctt ggtataaggc atctcatctc ctttctgtac ccgaggtaag 13680
   atgtgcaaga cacgggaact ccttatactt agctgaaggg agcggagcca tcatgagtct 13740
   tctcgaactg catgtaccac atgaaactat ctattacaat acgctctttt caaatgagat 13800
   gaaccccccg caacgacatt tcgggccgac cccaactcag tttttgaatt cggttgttta 13860
   taggaatcta caggcggagg taacatgcaa agatggattt gtccaagagt tccgtccatt 13920
   atggagagaa aatacagagg aaagtgacct gacctcagat aaagcagtgg ggtatattac 13980
   atctgcagtg ccctacagat ctgtatcatt gctgcattgt gacattgaaa ttcctccagg 14040
   gtccaatcaa agcttactag atcaactagc tatcaattta tctctgattg ccatgcattc 14100
   tgtaagggag ggcggggtag taatcatcaa agtgttgtat gcaatgggat actactttca 14160
   tctactcatg aacttgtttg ctccgtgttc cacaaaagga tatattctct ctaatggtta 14220
   tgcatgtcga ggagatatgg agtgttacct ggtatttgtc atgggttacc tgggcgggcc 14280
   tacatttgta catgaggtgg tgaggatggc aaaaactctg gtgcagcggc acggtacgct 14340
   cttgtctaaa tcagatgaga tcacactgac caggttattc acctcacagc ggcagcgtgt 14400
   gacagacatc ctatccagtc ctttaccaag attaataaag tacttgagga agaatattga 14460
   cactgcgctg attgaagccg ggggacagcc cgtccgtcca ttctgtgcgg agagtctggt 14520
   gagcacgcta gcgaacataa ctcagataac ccagattatc gctagtcaca ttgacacagt 14580
   tatccggtct gtgatatata tggaagctga gggtgatctc gctgacacag tatttctatt 14640
   taccccttac aatctctcta ctgacgggaa aaagaggaca tcacttatac agtgcacgag 14700
   acagatccta gaggttacaa tactaggtct tagagtcgaa aatctcaata aaataggcga 14760
   tataatcagc ctagtgctta aaggcatgat ctccatggag gaccttatcc cactaaggac 14820
   atacttgaag catagtacct gccctaaata tttgaaggct gtcctaggta ttaccaaact 14880
   caaagaaatg tttacagaca cttctgtatt gtacttgact cgtgctcaac aaaaattcta 14940
   catgaaaact ataggcaatg cagtcaaagg atattacagt aactgtgact cttaacgaaa 15000
   atcacatatt aataggctcc ttttttggcc aattgtattc ttgttgattt aatcatatta 15060
   tgttagaaaa aagttgaacc ctgactcctt aggactcgaa ttcgaactca aataaatgtc 15120
   ttaaaaaaag gttgcgcaca attattcttg agtgtagtct cgtcattcac caaatctttg 15180
   tttggt 15186
<210> 4
   <211> 1611
   <212> DNA
   <213> infectious bronchitis virus (IBV)
<400> 4
   atgttggtaa cacctctttt actagtgact cttttgtgtg cactatgtag tgctgctttg      60
   tatgacagta gttcttacgt gtactactac caaagtgcct tcagaccacc tgatggttgg     120
   catttacatg ggggtgcgta tgcggttgtt aatatttcta gtgaatctaa taatgcaggc     180
   tcttcatctg ggtgtactgt tggtattatt catggtggtc gtgttgttaa tgcttcttct     240
   atagctatga cggcaccgtc atcaggtatg gcttggtcta gcagtcagtt ttgtactgca     300
   tactgtaact tttcagatac tacagtgttt gttacacatt gttacaaaca tgttgggtgt     360
   cctataactg gcatgcttca acagcattct atacgtgttt ctgctatgaa aaatggccag     420
   cttttttata atttaacagt tagtgtagct aagtacccta cttttaaatc atttcagtgt     480
   gttaataatt taacatccgt atatttaaat ggtgatcttg tttacacctc taatgagacc     540
   acagatgtta catctgcagg tgtttatttt aaagctggtg gacctataac ttataaagtt     600
   atgagagaag ttagagccct ggcttatttt gttaatggta ctgcacaaga tgttattttg     660
   tgtgatgggt cacctagagg cttgttagca tgccagtata atactggcaa tttttcagat     720
   ggcttttatc cttttactaa tagtagttta gttaagcaga agtttattgt ctatcgtgaa     780
   aatagtgtta atactacttt tacgttacac aatttcactt ttcataatga gactggcgcc     840
   aacccaaatc ctagtggtgt ccagaatatt caaacttacc aaacacaaac agctcagagt     900
   ggttattata attttaattt ttcctttctg agtagttttg tttataagga gtctaatttt     960
   atgtatggat cttatcaccc aagttgtaat tttagactag aaactattaa taatggtttg    1020
   tggtttaatt cactttcagt ttcaattgct tacggtcctc ttcaaggtgg ttgcaagcaa    1080
   tctgtcttta gtggtagagc aacctgttgt tatgcttact catatggagg tcctttgctg    1140
   tgtaaaggtg tttattcagg tgagttagat cataattttg aatgtggact gttagtttat    1200
   gttactaaga gcggtggctc tcgtatacaa acagccactg aaccgccagt tataactcaa    1260
   cacaattata ataatattac tttaaatact tgtgttgatt ataatatata tggcagaact    1320
   ggccaaggtt ttattactaa tgtaaccgac tcagctgtta gttataatta tctagcagac    1380
   gcaggtttgg ctattttaga tacatctggt tccatagaca tctttgtcgt acaaagtgaa    1440
   tatggtctta attattataa ggttaaccct tgcgaagatg tcaaccagca gtttgtagtt    1500
   tctggtggta aattagtagg tattcttact tcacgtaatg agactggttc ccagcttctt    1560
   gagaatcagt tttacatcaa aatcactaat ggaacacgtc gttttagacg t             1611
<210> 5
   <211> 960
   <212> DNA
   <213> infectious laryngotracheitis virus (ILTV)
<400> 5
   ggaggggaga gagacaactt cagctcgaag tctgaagaga catcatgagc ggcttcagta     60
   acataggatc gattgccacc gtttccctag tatgctcgct tttgtgcgca tctgtattag    120
   gggcgccggt actggacggg ctcgagtcga gccctttccc gttcgggggc aaaattatag    180
   cccaggcgtg caaccgcacc acgattgagg tgacggtccc gtggagcgac tactctggtc    240
   gcaccgaagg agtgtcagtc gaggtgaaat ggttctacgg gaatagtaat cccgaaagct    300
   tcgtgttcgg ggtggatagc gaaacgggca gtggacacga ggacctgtct acgtgctggg    360
   ctctaatcca taatctgaac gcgtctgtgt gcagggcgtc tgacgccggg atacctgatt    420
   tcgacaagca gtgcgaaaaa gtgcagagaa gactgcgctc cggggtggaa cttggtagtt    480
   acgtgtctgg caatggatcc ctggtgctgt acccagggat gtacgatgcc ggcatctacg    540
   cctaccagct ctcagtgggt gggaagggat ataccgggtc tgtttatcta gacgtcggac    600
   caaaccccgg atgccacgac cagtatgggt acacctatta cagcctggcc gacgaggcgt    660
   cagacttatc atcttatgac gtagcctcgc ccgaactcga cggtcctatg gaggaagatt    720
   attccaattg tctagacatg cccccgctac gcccatggac aaccgtttgt tcgcatgacg    780
   tcgaggagca ggaaaacgcc acggacgagc tttacctatg ggacgaggaa tgcgccggtc    840
   cgctggacga gtacgtcgac gaaaggtcag agacgatgcc caggatggtt gtcttttcac    900
   cgccctctac gctccagcag tagccacccg agagtgtttt ttgtgagcgc ccacgcaaca    960
<210> 6
   <211> 810
   <212> DNA
   <213> avian encephalomyelitis virus (AEV)
<400> 6
   gggaaagagg atgaaggagg atttttcagt gtgcctgaag tggagcaaca tgttgttgag 60
   gataaggaac cacagggacc tttgcacgtg acaccttttg gcgctgttaa agctatggag 120
   gacccccaat tggccaggaa aacacctggc acattccctg aattagctcc tggtaaacct 180
   cgacatacag tggaccacat ggatctgtat aagttcatgg ggcgtgccca ttacttgtgg 240
   ggacatgaat tcaccaaaac tgacatgcag tacacattcc agataccatt aagtcccatt 300
   aaagagggtt ttgtgacggg tacacttagg tggtttttaa gtcttttcca actgtatcgt 360
   ggttctctcg acattaccat gacatttgca ggaaaaacta atgtggatgg cattgtgtac 420
   tttgtgcctg agggtgttgc gatagagact gagagggagg agcagacccc tttgctcaca 480
   ttgaactata aaacatcggt aggtgccatt aggtttaata ctggacaaac tacgaatgtc 540
   cagtttagga tccctttcta cacgccactg gaacacatcg caacccattc taaaaatgcg 600
   atggattcag tcttgggggc aatcacaacc cagatcacta actatagtgc tcaggatgag 660
   tatttgcagg ttacctacta catcagtttc aatgaagatt cacagttttc tgttcccaga 720
   gcggtgccag tggtcagctc attcactgac acatctagca aaacagtgat gaatacatat 780
   tggcttgatg atgacgagtt ggtagaagag 810
<210> 7
   <211> 726
   <212> DNA
   <213> avian encephalomyelitis virus (AEV)
<400> 7
   atgagcaaac tattttctac tgtaggcagg actgttgatg aggttttgtc tgtgctcaat      60
   gatgaggata ctgaatctta tgctggccct gatcgcactg cagtagttgg cggaggattt     120
   ctgacaacgg tagaccagag ttcagttagc acggctacaa tgggaagttt acaagatgta     180
   cagtacagga ctgcagtcga tattcctggt tctagagtga cacaaggtga gaggttcttc     240
   cttatcgatc agcgtgagtg gaactcaaca cagagtgaat ggcagttatt gggcaagatt     300
   gacatagtaa aagagctgct tgatcagtcg tatgctgttg atggcctttt gaagtaccat     360
   tcttatgcaa ggtttggctt ggatgtcatt gttcagatta atccaacatc attccaggca     420
   gggggcctca tagcagctct cgtaccttat gaccaggttg acattgaatc aattgttgcc     480
   atgaccactt attgccatgg caaggttaat tgcaacataa actacgttgt aaggatgaag     540
   gtgccatata tatacagtcg aggttgttac aaccttagga actcagcata ctccatttgg     600
   atgcttgtga taagagtgtg gtcacggctg cagttgggat ctggcacttc aacacagatt     660
   actatcacca ccttggctag gtttgtggat ttggaactgc atggacttag ccctttggtc     720
   gcacag                                                                726
<210> 8
   <211> 735
   <212> DNA
   <213> avian encephalomyelitis virus (AEV)
<400> 8
   atgatgcgca acgaatttcg actgtcgtca tctagcaaca ttgtcaattt ggctaattat      60
   gacgatgcaa gagccaaagt gtctctagcg ctgggacaag aagagttttc cagagactcg     120
   tcaagtaccg ggggggaatt ggtgcatcat ttttcacagt ggacgtccat tccgtgcctt     180
   gccttcactt ttacattccc cggcacggta gggccaggca ctcacatctg gtcaaccacg     240
   gtggaccctt tttcctgtaa cttgagggcg tctagcactg tgcaccccac taacttgagc     300
   tcgattgcgg gtatgttctg tttttggaga ggtgacattg tatttgagtt tcaagtcttt     360
   tgcaccaagt atcattccgg caggttgatg tttgtgtatg tgcctggcga tgaaaacaca     420
   aaaatcagca ccttaactgc aaaacaagca tctactggtc ttactgctgt ttttgatatc     480
   aatggtgtaa attcaacact ggtgtttaga tgccctttca tctctgacac accttacagg     540
   gtgaatccaa cgactcataa gtccctctgg ccttatgcaa ctggcaagct tgtgtgctat     600
   gtctacaata tactgaacgc acctgccagt gtatcaccaa ccctgcccat taatgtgtac     660
   aaaagtgctg cggatctgga gttgtatgca cctgtttatg gggtttctcc caccaacacc 720
   tcaatttttg ttcaa 735
<210> 9
   <211> 1500
   <212> DNA
   <213> avian parainfluenza virus (APV)
<400> 9
   ggggggtgtg catggtaggg tggggaaggt agccaattcc tgcccattgg gccgaccgta 60
   ccaagagaag tcaacagaag tatagatgca gggcgacatg gagggtagcc gtgataacct 120
   cacagtagat gatgaattaa agacaacatg gaggttagct tatagagttg tatccctcct 180
   attgatggtg agtgccttga taatctctat agtaatcctg acgagagata acagccaaag 240
   cataatcacg gcgatcaacc agtcgtatga cgcagactca aagtggcaaa cagggataga 300
   agggaaaatc acctcaatca tgactgatac gctcgatacc aggaatgcag ctcttctcca 360
   cattccactc cagctcaata cacttgaggc aaacctgttg tccgccctcg gaggttacac 420
   gggaattggc cccggagatc tagagcactg tcgttatccg gttcatgact ccgcttacct 480
   gcatggagtc aatcgattac tcatcaatca aacagctgac tacacagcag aaggccccct 540
   ggatcatgtg aacttcattc cggcaccagt tacgactact ggatgcacaa ggatcccatc 600
   cttttctgta tcatcatcca tttggtgcta tacacacaat gtgattgaaa caggttgcaa 660
   tgaccactca ggtagtaatc aatatatcag tatgggggtg attaagaggg ctggcaacgg 720
   cttaccttac ttctcaacag tcgtgagtaa gtatctgacc gatgggttga atagaaaaag 780
   ctgttccgta gctgcgggat ccgggcattg ttacctcctt tgtagcctag tgtcagagcc 840
   cgaacctgat gactatgtgt caccagatcc cacaccgatg aggttagggg tgctaacaag 900
   ggatgggtct tacactgaac aggtggtacc cgaaagaata tttaagaaca tatggagcgc 960
   aaactaccct ggggtagggt caggtgctat agcaggaaat aaggtgttat tcccatttta 1020
   cggcggagtg aagaatggat caacccctga ggtgatgaat aggggaagat attactacat 1080
   ccaggatcca aatgactatt gccctgaccc gctgcaagat cagatcttaa gggcagaaca 1140
   atcgtattat cctactcgat ttggtaggag gatggtaatg cagggagtcc taacatgtcc 1200
   agtatccaac aattcaacaa tagccagcca atgccaatct tactatttca acaactcatt 1260
   aggattcatc ggggcggaat ctaggatcta ttacctcaat ggtaacattt acctttatca 1320
   aagaagctcg agctggtggc ctcaccccca aatttaccta cttgattcca ggattgcaag 1380
   tccgggtacg cagaacattg actcaggcgt taacctcaag atgttaaatg ttactgtcat 1440
   tacacgacca tcatctggct tttgtaatag tcagtcaaga tgccctaatg actgcttatt 1500
<210> 10
   <211> 1440
   <212> DNA
   <213> hemorrhagic enteritis virus (HEV)
<400> 10
   gaaatgttaa tgttagacca tactgaccaa ttcctggttc attttagatg gaatcttcga      60
   acactgccac tagaattttt gctccaacgg aagggagaaa cagtataatt tacagcaact     120
   tgcctcctgt tcaagataca accaaaatat tttatataga taacaaggcc attgatatag     180
   agtcatataa tcaagagaaa gatcattcta attattatac taatataatt caaacacaga     240
   acatttcaac tattgattca agtatacagc aaattcagtt agatgaaagg tctagatggg     300
   gaggagaact acatacaagc ttagtaacat ctgttatgaa ttgtactaaa cattttaatt     360
   cagataggtg tttagtgaaa attcagacta ttaagagtcc acctacattt gaatggaaag     420
   aattgaaaat acctgaggga aactatgttt taaatgagtt tattgattta ttaaatgaag     480
   gtattacttc tttatacctt cagtatggca ggcaacaggg tgtacttgaa gaagacatag     540
   gaataaaatt tgatactcgc aattttgaaa ttggtaaaga tccaactact aatcttgtta     600
   ctcctggtaa atacttgttt aagggttatc atgctgatat aatacttctt cctggttggg     660
   ctattgattt ttctttttct agattgggta acattttagg tattagaaaa cgtgagactt     720
   ataaagctgg ctttttgatt gaatatgatg acttgacaaa tggtaatatt ccaccactgt     780
   tggatgttgc taactataag tctacaagtc aagctaaacc attattacag gatccatctg     840
   gcagatctta ccacgttatg gatagtgatt ctaacagacc tgtgactgca tataggtctt     900
   ttgttttgtc atataacaat gaaggtgctg caaaattaaa gtttttgatg tgtatgagtg     960
   atataacggg gggtctcaat cagctgtatt ggtgtttgcc tgattcttat aaaccgccag    1020
   tatcttttaa gcaagaaacg caagtagata aactgcctgt tgttggtatg caactttttt    1080
   tcctttttgt ttgtaaatct gtgtattctg gtgctgctgt ttacacacag ttaattgaac    1140
   agcagactaa tttgacacaa atttttaaca gatttcatga taatgaaatt ttaaaacaag    1200
   ctccatatgt gaatcaagtt ttattggctg aaaatgtgcc cataaatgtt aatcagggaa    1260
   caataccaat attttcaact cttccaggag tacagagagt ggttgtggaa gacgatagga    1320
   gaagaactgt accctacgtt accaagtcac ttgctacagt atatccgaag gttttgtcta    1380
   gcaaaacttt gcaataatgc attctgttgt ttattctcca ggggacagta gaggatgggg    1440
<210> 11
   <211> 2995
   <212> DNA
   <213> fowlpox virus (FPV)
<400> 11
   ctcttaattc gtttcaaaaa tgggaaatat ttttaagcct attccaaagg ccgattatca   60
   gattgtggaa acagtaccac aaagcttaac agctattaat tctactaatc tttctactta  120
   tgaatgtttt aaacgtttaa tagatctagc aaaaaaagag atctacatag ctacgttttg  180
   ttgtaaccta agtactaatc ctgagggtac tgacatacta aacagattaa tcgatgtttc  240
   gagtaaagtt tctgtatata ttttagtaga tgagagcagt cctcataaag attatgaaaa  300
   gattaagtct tcccatatta gttatattaa agtagatata ggtgtgctta ataatgaatc  360
   agtaggaaac ttgttaggta atttctgggt agtggataag cttcactttt atataggtag  420
   tgcgtctctt atgggaaatg cgctaacaac tattaaaaat atgggcatat attccgaaaa  480
   taattcttta gcaatggatt tatatttcag atcgttggac tataaaatta taagcaagaa  540
   aaaatgttta ttctttacca gaatggccac aaagtaccat ttcttcaaaa accataacgg  600
   tatattcttt tcagattctc cagaacatat ggtaggtaga aaaagaactt ttgacttgga  660
   ttgtgttatt cattatatag acgcggcgaa gtctactata gatctagcga tagtatctct  720
   tcttcctaca aagagaacaa aagattctat cgtctattgg cctataataa aagatgcatt  780
   aatacgggcc gtattagaac gaggtgtcaa actacgagtg ctattaggat tttggaaaaa  840
   aacggatgtt atatcaaaag catctataaa aagccttaac gaactaggag ttgaccatat  900
   agatatctct actaaagtat ttaggtttcc cgttaattct aaagtagatg atattaataa  960
   ttctaaaatg atgattatag atggaaggta tgctcatgtt atgactgcta acctagacgg 1020
   gtctcatttt aatcaccatg cttttgttag ctttaactgt atggatcaac aatttacaaa 1080
   gaaaatagct gaagtgtttg aaagggactg gatatctcct tacgcaaaag aaatagatat 1140
   gtctcaaata tagtatatat gataaaaaga tcctaataaa taaatatagc atggcactaa 1200
   tagaacagtt acaatcttct gaacaatcaa tactttcacc gtttagatat tatggtttta 1260
   aagattttca taatgtaatt tttaccacaa tagatgacga aacattaata gtaattacag 1320
   tcaacaatgt accattagta actaggttaa taacgtttga aaaaataaca ttttttagat 1380
   cgtttaatag tacttgtatt ataacttcca acaataattc ggatattgat acagatactt 1440
   attttatacc aaattcgtta tcactactag atattttgaa gaaaagagca tatgatgtag 1500
   aactaagaga tctatcattt gctataatgt cggaaatgaa taacgatgaa ttgagaaata 1560
   gtgatattgt atctctaaac aaatggctac ataagcataa tttactagac tacaaattag 1620
   tactaataag tgatatcgat agaagatata aattatacaa taaaaaaaat acaataattg 1680
   atgttatatc cgtaaatggt agaaattata atatatgggt taaagatgtt atagaatatt   1740
   attcaccgga atacttaaga tggtctatag atattaaaag agccacagaa agtaataact   1800
   ggttaccgta tagccagtct ataaaccctt tgaatgaaaa tatatacgct tttgaattta   1860
   tagctacttt agaaagatcc aatgagcgct taaatatcgg agcgatattc ctgtatccgg   1920
   atataataat tacaggtaga aacaacgaag atataataga aaagttttta gatcagttag   1980
   aagaagtaat atataaaaaa aattctgata gtattgtttt aacaggttat catctaacat   2040
   ttttagagaa tactatttta gagagatata tcagtaagta taaagactgg atttttacat   2100
   gtaatcgtct agtacattgt aaaaccggca ctgaagtatt cttatttgat gccgctatat   2160
   tttttccatc ctctaataag aaaggatatg taaaacattg gacaggtaaa aaattaaatt   2220
   ttaaaaactt tttccaaaaa gatagtcagc tagaaaaata cataaataat aacagtgtag   2280
   cagaacgtat atattattta cagtcttctt tacacaagca tatatcctgt ctaatagaaa   2340
   ttttcgagtt aaatggattt gattttaatt tttctgggtt gttagatata cttattttca   2400
   gtattcgtgt taagaataat aatggtaatt actattaccc taaacattct tcagctgtga   2460
   atttgatgtt gtcatctatt tacacggact attatgctat tgatgatata gataaagata   2520
   gtaagaaact tgtttttaac tctatttttc ctttaataat ggaaggatat taccctgaag   2580
   gaaaacctta ttatacgaaa acacccaaag aagggtattt gtcaatatgt ttatgtgatg   2640
   tagaaatatc taatgatata aagaatccta tattgtattg taaagaaaac aagtcagcta   2700
   ggaagtttac aggagtattc acatctgtag atatagatac cgctgtaaaa ctaagaggat   2760
   ataaaattaa aatattagaa tgtattgaat ggcctaataa aataaaatta ttcgacaata   2820
   tatgttatct gaataaatta tttatagaac atcaggatta cacacacgat gaaaaatctt   2880
   tacaaggcta tcttttttct tatttactta aaggcaacgt taccgaagat gttttagcta   2940
   tgaaaagttg tagaaataat ctttctataa tatcatttat aataagttac tgcag        2995
<210> 12
   <211> 35
   <212> DNA
   <213> artificial sequence
<220>
   <223> PCR primer, IBS1F'
<400> 12
   cgggatccgc cgccgccatg ttggtaacac ctctt 35
<210> 13
   <211> 29
   <212> DNA
   <213> artificial sequence
<220>
   <223> PCR primer, IBS1R'
<400> 13
   cggaattctt aacgtctaaa acgacgtgt 29
<210> 14
   <211> 38
   <212> DNA
   <213> artificial sequence
<220>
   <223> PCR primer, NDF F'
<400> 14
   cgggatccgc cgccgccatg ggctccagac cttctacc 38
<210> 15
   <211> 34
   <212> DNA
   <213> artificial sequence
<220>
   <223> PCR primer, NDF R'
<400> 15
   ccgctcgagt tacatttttg tagtggctct catt 34
<210> 16
   <211> 39
   <212> DNA
   <213> artificial sequence
<220>
   <223> PCR primer, NDHN F'
<400> 16
   cgggatccgc cgccgccatg gaccgcgccg ttaggcaag 39
<210> 17
   <211> 29
   <212> DNA
   <213> artificial sequence
<220>
   <223> PCR primer, NDHN R'
<400> 17
   gctctagatt actcaactag ccagacctg 29
<210> 18
   <211> 35
   <212> DNA
   <213> artificial sequence
<220>
   <223> PCR primer, IBDVP2F'
<400> 18
   cgggatccgc cgccgccatg acaaacctgc aagat 35
<210> 19
   <211> 29
   <212> DNA
   <213> artificial sequence
<220>
   <223> PCR primer, IBDVP2R'
<400> 19
   cggaattctt accttatggc ccggattat 29

## Description

### RELATED APPLICATION:

This application claims the priority of U.S. Provisional Application Serial No. 60/362,547, filed on March 8, 2002.

### FIELD OF THE INVENTION:

The present invention relates to either a multiple DNA vaccine or a multivalent DNA vaccine for use in acquiring embroyonic immunity in fowl eggs and methods for preparing and using the same. The multiple DNA vaccine contains two or more DNA constructs, each containing a DNA molecule encoding an avian viral protein or a fragment thereof capable of inducing a protective immune response against an avian viral disease in fowl. The multivalent DNA vaccine contains a DNA construct which contains two or more DNA molecules. Each of the DNA molecules represents an avian viral gene or a fragment thereof. The multivalent DNA vaccine is capable of expressing two or more viral antigens and inducing protective immune responses against two or more of the avian viral diseases in fowl. Both the multiple DNA vaccine and the multivalent DNA vaccine are preferred to be injected into the amniotic fluid of the fowl egg after being fertilized for about 18 days.

### BACKGROUND OF THE INVENTION:

*In ovo* vaccination of virus-containing vaccines was extensively described by Sharma et al. (U.S. Pat. No. 4,458,630). In particular, it teaches that live Marek's disease virus can be injected into amniotic fluid within the egg, whereafter the embryo is infected and the vaccine virus replicates to a high titer which induces the formation of protective antibodies in the treated embryo. (See Sharma (1985), Avian Diseases 29, 1155, 1167-68).

It is well-known in the worldwide poultry business that certain viral diseases, such as Marek's disease virus (MDV), infectious bursal disease virus (IBDV), Newcastle disease virus (NDV), infectious bronchitis virus (IBV), infectious laryngotracheitis virus (ILTV), avian encephalomyelitis (AEV), chick anemia virus (CAV), Fowlpox virus (FPV), avian influenza virus (AIV), reovirus, avian leukosis virus (ALV), reticuloendotheliosis virus (REV), avian adenovirus and hemorrhagic enteritis virus (HEV), may cause major outbreak and result in significant economic losses in the commercial poultry industry. Among them, MDV, IBDV, NDV and IBV, are particularly important due to their virulent nature.

Marek's Disease (MD) is a malignant, lymphoproliferative disorder disease that occurs naturally in chickens. The disease is caused by a herpesvirus: Marek's Disease Virus (MDV). MD is ubiquitous, occurring in poultry-producing countries throughout the world. Chickens raised under intensive production systems will inevitably suffer losses from MD. The symptoms of MD appear widely in the nerves, genital organs, internal organs, eyes and skin of the infected birds, causing motor trouble (due to paralysis when the nerves have been affected), functional trouble of the internal organs (due to tumors), and chronic undernourishment (if the internal organs are attacked by the virus). MD affects chickens from about 6 weeks of age, occurring most frequently between ages of 12 and 24 weeks.

At of this time, there are no methods of treating MD. The control of the disease is based primarily on management methods such as insolating growing chickens from sources of infection, the use of genetically resistant stock, and vaccination. However, management procedures are normally not cost-effective and the progress has been disappointing with respect to the selection of poultry stock with increased genetically controlled resistance. Nowadays, control of MD is almost entirely based on vaccination.

Infectious bursal disease virus (IBDV) is responsible for a highly contagious immunosuppressive disease in young chickens which causes significant losses to the poultry industry worldwide (See Kibenge (1988), J. Gen. Virol., 69:1757-1775). Infection of susceptible chickens with virulent IBDV strains can lead to a highly contagious immunosuppressive condition known as infectious bursal disease (IBD). Damage caused to the lymphoid follicles of the bursa of Fabricius and spleen can exacerbate infections caused by other agents and reduce a chicken's ability to respond to vaccination as well (See Cosgrove (1962), Avian Dis., 6:385-3894).

IBDV is a member of the Birnaviridae family and its genome consists of two segments of double-stranded RNA (See Dobos et al (1979), J. Virol., 32:593-605). The smaller segment B (about 2800 bp) encodes VP1, the dsRNA polymerase. The larger genomic segment A (about 3000 bp) encodes a 110 kDa precursor polypeptide in a single open reading frame (ORF) that is processed into mature VP2, VP3 and VP4 (See Azad et al (1985), Virology, 143:35-44). From a small ORF partly overlapping with the polypeptide ORF, segment A can also encode VP5, a 17-kDa protein of unknown function (See Kibenge et al (1991), J. Gen. Virol. 71:569-577).

While VP2 and VP3 are the major structural proteins of the virion, VP2 is the major host-protective immunogen and causes induction of neutralizing antibodies (See Becht et al. (1988), J. Gen. Virol., 69:631-640; Fahey et al. (1989), J. Gen. Virol., 70:1473-1481). VP3 is considered to be a group-specific antigen because it is recognized by monoclonal antibodies (Mabs) directed against VP3 from strains of both serotype 1 and 2 (See Becht et al (1988), J. Gen. Virol., 69:631-640). VP4 is a virus-coded protease and is involved in the processing of the precursor protein (See Jagadish et al. (1988), J. Virol., 62:1084-1087).

In the past, control of IBDV infection in young chickens has been achieved by live vaccination with avirulent strains, or principally by the transfer of maternal antibody induced by the administration of live and killed IBDV vaccines to breeder hens. Unfortunately, in recent years, virulent variant strains of IBDV have been isolated from vaccinated flocks in the United States (See e.g., Snyder et al. (1988), Avian Dis., 32:535-539; Van der Marel et al. (1990), Dtsch. Tierarztl. Wschr., 97:81-83), which drastically undermine the effectiveness of using live vaccination for IBDV.

Efforts to develop a recombinant vaccine for IBDV have also been made, and the genome of IBDV has been cloned (See Azad et al (1985) "Virology", 143:35-44). The VP2 gene of IBDV has been cloned and expressed in yeast (See Macreadie et al. (1990), Vaccine, 8:549-552), as well as in recombinant fowlpox virus (See Bayliss et al (1991), Arch. Virol., 120:193-205). When chickens were immunized with the VP2 antigen expressed from yeast, antisera afforded passive protection in chickens against IBDV infection. When used in active immunization studies, the fowlpox virus-vectored VP2 antigen afforded protection against mortality, but not against damage to the bursa of Fabricius.

Newcastle disease virus (NDV) is an enveloped virus containing a linear, single-strand, nonsegmented, negative sense RNA genome. Typically, virus families containing enveloped single-stranded RNA of the negative-sense genome are classified into groups having non-segmented genomes (e.g., Paramyxoviridae and Rhabdoviridae) or those having segmented genomes (e.g., Orthomyxoviridae, Bunyaviridae and Arenaviridae). NDV, together with parainfluenza virus, Sendai virus, simian virus 5, and mumps virus, belongs to the Paramyxoviridae family.

The structural elements of the NDV include the virus envelope which is a lipid bilayer derived from the cell plasma membrane. The glycoprotein, hemagglutinin-neuraminidase (HN) protrude from the envelope allowing the virus to contain both hemagglutinin and neuraminidase activities. The fusion glycoprotein (F), which also interacts with the viral membrane, is first produced as an inactive precursor, then cleaved post-translationally to produce two disulfide linked polypeptides. The active F protein is involved in penetration of NDV into host cells by facilitating fusion of the viral envelope with the host cell plasma membrane. The matrix protein (M), is involved with viral assembly, and interacts with both the viral membrane as well as the nucleocapsid proteins.

The main protein subunit of the NDV nucleocapsid is the nucleocapsid protein (NP) which confers helical symmetry on the capsid. In association with the nucleocapsid are the P and L proteins. The phosphoprotein (P), which is subject to phosphorylation, is thought to play a regulatory role in transcription, and may also be involved in methylation, phosphorylation and polyadenylation. The L gene, which encodes an RNA-dependent RNA polymerase, is required for viral RNA synthesis together with the P protein. The L protein, which takes up nearly half of the coding capacity of the viral genome is the largest of the viral proteins, and plays an important role in both transcription and replication.

The replication of all negative-strand RNA viruses, including NDV, is complicated by the absence of cellular machinery required to replicate RNA. Additionally, the negative-strand genome can not be translated directly into protein, but must first be transcribed into a positive-strand (mRNA) copy. Therefore, upon entry into a host cell, the virus can not synthesize the required RNA-dependent RNA polymerase. The L, P and NP proteins must enter the cell along with the genome on infection. Both the NDV negative strand genomes (vRNAs) and antigenomes (cRNAs) are encapsidated by nucleocapsid proteins; the only unencapsidated RNA species are virus mRNAs. The cytoplasm is the site of NDV viral RNA replication, just as it is the site for transcription. Assembly of the viral components appears to take place at the host cell plasma membrane and mature virus is released by budding.

In U.S. Pat. No. 5,427,791, Ahmad et al. describe the embryonal vaccination against NDV, which requires the modification of the viruses through the use of ethyl methane sulfonate (EMS). However, EMS is a mutagen so that the vaccine prepared by the use of EMS is suspected to act as a mutagen as well, which is undesirable for regular administration of the vaccine. Nevertheless, without the modification with EMS, the NDV vaccine cannot be applied for *in ovo* vaccination as almost all of the embryos will die upon injection of the eggs with the unmodified virus.

Infectious bronchitis virus (IBV), the prototype of the family Coronaviridae, is the etiological agent of infectious bronchitis (IB). The virus has a single-stranded RNA genome, approximately 20 kb in length, of positive polarity, and is usually about 80-100 nm in size, being round with projecting 20 nm spikes. IBV is the causative agent of an acute, highly contagious disease in chickens of all ages, affecting the respiratory, reproductive and renal systems.

IBV contains three structural proteins: the spike (S) glycoprotein, the membrane glycoprotein, and the nucleocapsid protein. The spike glycoprotein is so called because it is present in the teardrop-shaped surface projections or spikes protruding from the lipid membrane of the virus. The spike protein is believed likely to be responsible for immunogenicity of the virus, partly by analogy with the spike proteins of other corona-viruses and partly by in vitro neutralisation experiments (See, e.g., D. Cavanagh et al. (1984), Avian Pathology, 13, 573-583). There are two spike glycoproteins, which are S1 (90,000 daltons) and S2 (84,000 daltons). The polypeptide components of the glycopolypeptides S1 and S2 have been estimated after enzymatic removal of oligosaccharides to have a combined molecular weight of approximately 125,000 daltons. It appears that the spike protein is attached to the viral membrane by the S2 polypeptide.

IBV has been wide-spread in countries where an intensive poultry industry has been developed. Young chickens up to 4 weeks of age are most susceptible to IBV, infection leading to high rates of morbidity and to mortality resulting from secondary bacterial infection. Infection also results in a drop in egg production, or failure to lay at full potential, together with an increase in the number of down-graded eggs with thin, misshapen, rough and soft-shells produced, which can have a serious economic effect.

Administering live vaccines to a developing chick in the egg (*in-ovo*) has proven to be a fast (40,000 eggs per hour), effective (100% of the eggs receive the vaccine), and labor saving ($100,000 per year per hatchery) method to vaccinate baby chicks against certain diseases before they hatch.

The first *in-ovo* vaccination machine for use on chicken hatching eggs was developed by Embrex, Inc., of Raleigh, N.C. in the late 1980s. (See U.S. Pat. Nos. 5,056,464 and 5,699,751). This in-ovo machine is currently used in about 80% of the U.S. broiler hatcheries, primarily for administering MD vaccines. The popularity of this machine, which has proven to be safe and effective in vaccination of chicks against MD, is also being used increasingly to administer IBD vaccines and ND vaccines.

In the invention to be presented in the following sections, a DNA-mediated immunization (collectively "DNA vaccines") will be introduced. There are two kinds of DNA vaccines, i.e., a multiple DNA vaccine and a multivalent DNA vaccine. The multiple DNA vaccine of the present invention contains a combination of two or more DNA construct, each containing a single DNA molecule which is a viral gene or a fragment thereof. The multivalent DNA vaccine of the present invention contains two or more viral genes or fragments thereof linking together in one DNA construct. The viral genes or fragments used in preparation of either the multiple DNA vaccine or the multivalent DNA vaccine are those that encode viral peptides which are antigenic to and can induce both the humoral and the cellular immune system in a host. The DNA vaccines are preferably applied to the egg by needles. The inj ection of the DNA vaccines *in ovo* leads to surprisingly strong immune responses which include not only antibody induction and T-cell activation with cytokine secretion, but also the production of cytotoxic T lymphocytes (CTL).

US Patent Application No. US19950477459 describes recombinant fowlpox viruses and the use in live vaccine to protect fowl against Newcastle disease virus and fowlpox virus.

Karaca et al., Vaccine. 1998 Oct;16(16):1496-503, disclose the influence of IFN on protective efficacy and humoral responses of chickens following *in ovo* or post-hatch administration of recombinant viruses by the recombinant fowlpox viruses which co-express chicken type I IFN and Newcastle disease virus HN and F genes.

McMillen et al., Dev Biol Stand. 1994;82:137-45, describe the safe and effective use of fowlpox virus as a vector for poultry vaccines.

WO 98/56413 describes multivalent *in ovo* avian vaccine.

Gagic et al., Avian Dis. 1999 Apr-Jun;43(2):293-301, disclose *in ovo* vaccination of specific-pathogen-free chickens with vaccines containing multiple agents.

### SUMMARY OF THE INVENTION:

The present invention provides a multiple DNA vaccine for in *ovo* injection. The multiple DNA vaccine contains two or more DNA constructs, each DNA construct expressing an antigenic protein of an avian virus causing avian viral disease in fowl. The antigenic protein of the avian virus is capable of inducing a protective immune response against an avian viral disease. The multiple DNA vaccine is preferred to inject into the egg, particularly the amniotic fluid of the egg, of the fowl. The egg is preferred to be fertilized for about 18 days. The preferred fowl includes chicken, turkey, duck, and goose.

The DNA construct contains a DNA molecule and a vector. The vector can be a plasmid or a viral carrier. The preferred vector is a plasmid. Examples of the plasmid include, but are not limited to, pcDNA3, pVAX1, pSectag, pTracer™, pDisplay™, pUC system plasmid (such as pUC7, pUC8, pUC18), and pGEM® system plasmid. Alternatively, any plasmid which contains a promoter such as CMV promoter, SV40 promoter, RSV promoter, and β-action promoter, can also be used for preparing the DNA construct. The most favorable plasmid is pcDNA3. The preferred viral carrier is one selected from the group consisting of a baculovirus, a herpes virus, and a pox virus.

Examples of the avian virus include, but are not limited to Marek's disease virus (MDV), infectious vursal disease virus (IBDV), Newcastle disease virus (NDV), infectious bronchitis virus (IBV), infectious laryngotracheitis virus (ILTV), avian encephalomyelitis (AEV), avian leukosis virus (ALV), fowlpox virus (FPV), avian paramyxovirus (APV), duck hepatitis virus (DHV), and hemorrhagic enteritis virus (HEV).

The DNA molecules that are particularly suitable for inducing a protective immune response against the avian viral diseases as shown above include, but are not limited to, the I entire of gB gene of Merk's Disease virus (MDV) having the DNA sequence of SEQ ID NO:1 or a fragment thereof; the entire VP2 gene of infectious bursal disease virus (IBDV) having the DNA sequence of SEQ ID NO:2 or a fragment thereof; the entire HN gene of Newcastle disease virus (NDV) having the DNA sequence (which is from bases 6321 to 8319) of SEQ ID NO:3 or a fragment thereof (i.e., SEQ ID NO:3 is the entire genome of the NDV); the entire S1 gene of infectious bronchitis virus (IBV) having the DNA sequence of SEQ ID NO:4 or a fragment thereof; the entire glycoprotein G gene of infectious laryngotracheitis virus (ILTV) having the DNA sequence of SEQ ID NO:5 or a fragment thereof; the entire VP1, VP0, or VP3 gene of avian encephalomyelitis virus (AEV) or a fragment therof (the VP1 gene has the DNA sequence of SEQ ID NO:6; the VP0 gene has the DNA sequence of SEQ ID NO:7; and the VP3 gene has the DNA sequence of SEQ ID NO:8); the entire paraglycoprotein G gene of avian parainfluenza virus (APV) having the DNA sequence of SEQ ID NO:9 or a fragment thereof; the entire type A penton base gene of hemorrhagic enteritis virus (HEV) having the DNA sequence of SEQ ID NO:10 or a fragment thereof; and the entire envelope antigen gene of fowlpox virus (FPV) having the DNA sequence of SEQ ID NO:11 or a fragment thereof.

One preferred example of the DNA vaccine contains two DNA constructs, each containing a DNA molecule capable of expressing a gene or a fragment thereof which is from Marek's disease virus (MDV), infectious vursal disease virus (IBDV), Newcastle disease virus (NDV), or infectious bronchitis virus (IBV).

Another preferred example of the multiple DNA vaccine contains three or more DNA constructs, each containing a DNA molecule capable of expressing a gene or a fragment thereof which is from Marek's disease virus (MDV), infectious vursal disease virus (IBDV), Newcastle disease virus (NDV), or infectious bronchitis virus (IBV).

The present invention also provides a method for vaccinating fowl egg and a method for preparing the multiple DNA vaccine. The method for vaccinating fowl egg includes injecting into the fowl egg the multiple DNA vaccine as shown above. The method for preparing the multiple DNA vaccine includes ligating a DNA molecule to a plasmid or virus carrier to form a DNA construct; and then mixing two or more said DNA constructs to form the multiple DNA vaccine. The insertion of the DNA molecule into the vector can be achieved by conventional method, i.e., by ligation the DNA molecule with an enzyme such as T4 DNA ligase when both the genes and the desired vector have been cut with the same restriction enzyme(s) as complementary DNA termini are thereby produced. For pcDNA3, the preferred restriction enzymes are BamH1 and EcoR1.

In another aspect there is provided a multivalent DNA vaccine for *in ovo* injection. The multivalent DNA vaccine comprises a DNA construct containing two or more DNA molecules linked together with a vector. Each of the DNA molecules expresses an antigenic protein of an avian virus, which is capable of inducing a protective immune response against that avian viral disease in fowl. The multivalent DNA vaccine is preferred to be injected into a fowl egg. Each of the DNA molecules of the multivalent DNA vaccine is a gene or a fragment thereof from Marek's disease virus (MDV), infectious vursal disease virus (IBDV), Newcastle disease virus (NDV), infectious bronchitis virus (IBV), infectious laryngotracheitis virus (ILTV), avian encephalomyelitis (AEV), avian leukosis virus (ALV), fowlpox virus (FPV), avian parainfluenza virus (APV), duck hepatitis virus (DHV), and hemorrhagic enteritis virus (HEV).

### DETAILED DESCRIPTION OF THE INVENTION:

Traditional avian vaccines comprise chemically inactivated virus vaccines or I modified live-virus vaccines. Inactivated vaccines require additional immunizations which are not only expensive to produce but also laborious to administer. Further, some infectious virus particles may survive the inactivation process and may cause disease after administration to the animal.

In general, attenuated live virus vaccines are preferred over inactivated vaccines because they evoke an immune response often based on both humoral and cellular reactions. Such vaccines are normally based on serial passage of virulent strains in tissue culture. However, the attenuation process induces mutations of the viral genome, resulting in a population of virus particles heterogeneous with regard to virulence and immunizing properties. In addition, it is well known that the traditional attenuated live virus vaccines can revert to virulence resulting in disease outbreaks in inoculated animals and the possible spread of the pathogen to other animals.

Thus, it is advantageous for the industry to employ vaccines based on recombinant DNA technology. The resulting DNA vaccines only contain and express the necessary and relevant immunogenic material that is capable of eliciting a protective immune response against the pathogens and would not display above mentioned disadvantages of the live or inactivated vaccines.

For the purpose of preparing multiple DNA vaccines or multivalent recombinant DNA vaccines, the DNA sequence of the gene (also used interchangeably as "DNA molecule") need not contain the full length of DNA encoding the polypeptides. In most cases, a fragment of the gene which encodes an epitope region should be sufficient enough for immunization. The DNA sequence of an epitope region can be found by sequencing the corresponding part of other viral strains and comparing them. The major antigenic determinants are likely to be those showing the greatest heterology. Also, these regions are likely to lie accessibly in the conformational structure of the proteins. One or more such fragments of genes encoding the antigenic determinants can be prepared by chemical synthesis or by recombinant DNA technology. These fragments of genes, if desired, can be linked together or linked to other DNA molecules.

Also, the viral genes need not be in DNA. In fact, some of the frequently found avian viral diseases are caused by double- or single-stranded RNA viruses. For example, Marek's diesease virus is a double-stranded RNA virus, while infectious bursal disease virus (IBDV), Newcastle disease virus (NDV) and infectious bronchitis virus (IB) are single-stranded RNA viruses. The RNA viral sequences, however, can be reverse-transcribed into DNA using RT-Polymerase chain reaction (RT-PCR) technology and then incorporated into a vector by the conventional recombinant DNA technology.

In addition, because of the degeneracy of the genetic code it is possible to have numerous RNA and DNA sequences that encode a specified amino acid sequence. Thus, all RNA and DNA sequences which result in the expression of a polypeptide having the antibody binding characteristics are encompassed by this invention.

To construct a recombinant DNA vaccine, either univalent or multivalent, the DNA sequence of the viral gene can be ligated to other DNA molecules with which it is not associated or linked in nature. Optionally, the DNA sequence of a viral gene can be ligated to another DNA molecule, i.e., a vector, which contains portions of its DNA encoding fusion protein sequences such as β-galactosidase, resulting in a so-called recombinant nucleic acid molecule or DNA construct, which can be used for transformation of a suitable host. Such vector is preferably derived from, e.g., plasmids, or nucleic acid sequences present in bacteriophages, cosmids or viruses.

Specific vectors which can be used to clone nucleic acid sequences according to the invention are known in the art and include either a plasmid or a virus carrier. Examples of the plasmid include, but are not limited to, pBR322, pcDNA3, pVAX1, pSectag, pTracer™, pDisplay™, pUC system plasmids (e.g., pUC7, pUC8, pUC18), pGEM® system plasmids, Bluescript plasmids or any other plasmids where CMV promoter, SV40 promoter, RSV promoter, or β-action promoter is included. The preferred plasmid is pcDNA3. Examples of the virus carrier include, but are not limited to, bacteriophages (e.g., λ and the M13-derived phages), SV40, adenovirus, polyoma, baculoviruses, herpes viruses (HVT) or pox viruses (e.g., fowl pox virus).

The methods to be used for the construction of a recombinant nucleic acid molecule are known to those of ordinary shill in the art. For example, the insertion of the nucleic acid sequence into a cloning vector can easily be achieved by ligation with an enzyme such as T4 DNA ligase when both the genes and the desired cloning vehicle have been cut with the same restriction enzyme(s) so that complementary DNA termini are thereby produced.

Alternatively, it may be necessary to modify the restriction sites so as to produce blunt ends either by digesting the single-stranded DNA or by filling in the recessive termini with an appropriate DNA polymerase. Subsequently, blunt end ligation with an enzyme such as T4 DNA ligase may be carried out. If desired, any restriction site may be produced by ligating linkers onto the DNA termini. Such linkers may comprise specific oligonucleotide sequences that encode restriction site sequences. The restriction enzyme cleaved vector and nucleic acid sequence may also be modified by homopolymeric tailing.

The present invention provides two kinds of DNA vaccines. The first kind is a multiple DNA vaccine, which includes two or more of univalent DNA vaccines, each containing a DNA sequence encoding at least one polypeptide affording protection against one viral disease such as Marek's dosease voris (MDV), infectious bursal disease virus (IBDV), Newcastle disease virus (NDV), infectious bronchitis virus (IBV), infectious laryngotracheitis virus (ILTV), avian encephalomyelitis (AEV), Fowlpox virus (FPV), avian influenza virus (AIV), avian leukosis virus (ALV), duck hepatitis virus B genome, and hemorrhagic enteritis virus (HEV), inserted into a commercially available plasmid.

The second kind is a multivalent recombinant DNA vaccine, which contains two or more genes or gene fragments from different viruses. These genes or gene fragments are carried by a useful vector, which can be either a plasmid or a virus carrier. The multivalent recombinant DNA vaccine encodes two or more antigenic polypeptides which afford protection against at least two viral diseases including, but not limited to, MD, IBD, ND or IB. The viral genes or gene fragments are operatively attached to the vector in reading frame so that they can be expressed in a host. The different structural DNA sequences carried by the vector may be separated by termination and start sequences so that the proteins can be expressed separately or they may be part of a single reading frame and therefore be produced as a fusion protein by methods known in the art.

The preferred DNA sequences include, but are not limited to, the entire of gB gene of Merk's Disease virus (MDV) having the DNA sequence of SEQ ID NO:1 or a fragment thereof; the entire VP2 gene of infectious bursal disease virus (IBDV) having the DNA sequence of SEQ ID NO:2 or a fragment thereof; the entire HN gene of Newcastle disease virus (NDV) having the DNA sequence of SEQ ID NO:3 or a fragment thereof; the entire S1 gene of infectious bronchitis virus (IBV) having the DNA sequence of SEQ ID NO:4 or a fragment thereof.

The DNA sequence encoding the gB polypeptide of MDV has the nucleic acid sequence as SEQ ID NO:1. The DNA sequence contains 3650 bp of linear DNA.

The DNA sequence encoding the VP2 polypeptide of IBDV has the nucleic acid sequence as SEQ ID NO:2. The DNA sequence contains 3004 bp of linear DNA molecule I which is reversely transcribed from IBDV's RNA template.

The DNA sequence of the entire genome ofNDV contains 15186 bps of DNA, wherein (1) base No. 56 to 1792 encodes NP polypeptide, which is nucleocapsid protein; (2) base No. 1804-3244 encodes P polypeptide, which is a phosphoprotein; (3) base No. 3256-4487 encodes M polypeptide, which is a matrix protein; (4) base No. 4498-6279 encodes F polypeptide, which is a fusion protein; (5) base 6321-8319 encodes HN polypeptide, which is a hemagglutinin-neuraminidase; (6) base No. 8370-15073 encodes L polypeptide, which is a large polymerase protein. The NDV genome has the DNA sequence as SEQ ID NO:3.

The DNA sequence of the S1 polypeptide contains 1611 bp of linear DNA sequence as shown in SEQ ID NO:4, which is reversely transcribed from IBV's RNA templates.

The following experimental designs are illustrative, but not limiting the scope of the present invention. Reasonable variations, such as those occur to reasonable artisan, can be made herein without departing from the scope of the present invention.

### I. Materials and Methods

### (A) Virus and vaccines

Avian infectious bronchitis virus (IBV), infectious bursal disease (IBD) and Newcastle disease (ND) vaccines were purchased from Intervet Inc.

### (B) Viral RNA isolation and RT-PCR

Two hundred microliter recovered attenuated vaccines (Intervet Inc.) were resolved in iced cold GTC buffer (4 M guanidium isothiocyanate, 25mM sodium citrate, pH 7.0, 0.5% Sarkosyl, 0.1 M - mercaptoethanol) and sodium acetate (pH 4). An equal volume of phenol-chloroform (1:1) was added-and placed on ice for 15 minutes after vortexing. The aqueous phase was collected after centrifuge and the RNA was precipitated with an equal volume of isopropanol. RNA was pelleted by centrifugation at 12000 rpm for 20 min at 4□ and then suspended in diethylpyrocarbonate (DEPC) treated deionized distill water and stored at -70□.

### (C) Oligonucleotides

Oligonucleotide primers for RT-PCR amplification were purchased from Promega, and were designed according to the genome of the Avian infectious bronchitis virus (Beaudette CK strain), Newcastle disease virus (Lasota strain) and Infectious bursa disease virus respectively. The sequences of the primers used for PCR were :
IBS1F' 5' CGGGATCCGCCGCCGCCATGTTGGTAACACCTCTT 3'; (SEQ ID NO:12)
IBS1R' 5' CGGAATTCTTAACGTCTAAAACGACGTGT 3'; (SEQ ID NO:13)
NDF F' 5' CGGGATCCGCCGCCGCCATGGGCTCCAGACCTTCTACC 3'; (SEQ ID NO:14)
NDF R' 5' CCGCTCGAGTTACATTTTTGTAGTGGCTCTCATT 3'; (SEQ ID NO:15)
NDHN F' 5' CGGGATCCGCCGCCGCCATGGACCGCGCCGTTAGGCAAG 3'; (SEQ ID NO:16)
NDHN R' 5' GCTCTAGATTACTCAACTAGCCAGACCTG 3'; (SEQ ID NO:17)
IBDVP2F' 5' CGGGATCCGCCGCCGCCATGACAAACCTGCAAGAT 3'; (SEQ ID NO:18)
IBDVP2R' 5' CGGAATTCTTACCTTATGGCCCGGATTAT 3'. (SEQ ID NO:19)

### (D) Reverse transcription polymerase reaction (RT-PCR)

Reverse transcription of IBV, NDV and IBDV RNA were carried out at 42□ for 30 min in 2.5 x *Taq* buffer (200 mM NaCl, 15 mM Tris-HCl, PH7.4, 15 mM MgCl₂, 15 mM β-mercaptoethanol, and 0.25 mM each of dATP, dCTP, dGTP, and dTTP). In addition to the *Taq* buffer, the reaction mixture (40 µl) also contained viral RNA, 2.4 U of avian myeloblastosis virus (AMV) reverse transcriptase (Promega), 16 U of RNasin (Promega), and 0.01 nmol reverse primer (*IBDVP2R, NDF F, NDHNF or IBS1R*). The final volume of the reaction mixture was 40 µl. After reverse transcription, the following reagents were added to the reverse transcription mixture: 0.02 nmol of each nucleotide triphosphate (dATP, dCTP, dGTP, dTTP), 0.01 nmol of forward primer (*IBDVP2F, NDF R, NDHN R or IBS1F*) and 1.5 U of *Taq* DNA polymerase (Strategene). Water was then added to a final volume of 100 µl. The reaction was carried out for 32 cycles in a Thermal Cycler (Perkin Elmer-Cetus). Each PCR cycle consisted of 1 min of denaturation at 94□, 1 min of annealing at 57□, and 2 min of DNA chain elongation at 72□.

### (E) Preparation of DNA constructs

The plasmids pCMV-VP2, pCMV-S1, pCMV-NDF and pCMV-NDHN were constructed with the VP2, S1, NDF and NDHN genes from IBD vaccine, IBV vaccine and NDV vaccine respectively, placed downstream of the commercial plasmid pcDNA3. (Invitrogen, U.S.A.). All of the genes were inserted into the pcDNA3 vector using restriction enzymes *Bam*H1*, EcoRl, Xba*I and XhoI (underlined characters in the sequence of the primers). Sequences of the all genes in the pcDNA3 vector were verified by sequencing in both directions.

### (F) Preparation of DNA and DNA delivery

The quantity of plasmid DNA that had been purified by affinity chromatography (Qiagen. Inc.) was determined by spectrophotometric measurements at 260 and 280 nm. The DNA in aliquots to 100 µg was suspended in 100 µl of PBS (0.14M NaCl, 10mM sodium phosphate, pH 7.4). For DNA delivery, 1cc syringe with a 20 gauge 1 and 1/2 inch needle were used. For the *in-ovo* groups, the embryos (18-day-old fertilized and developing eggs from the setting trays) were injected with 0.1 milliliters of DNA vaccine (100 µg) into the large end of each egg through the air cell with a needle. The eggs were then transferred into the hatchery where they remained until they hatched at about 21 days of age. For the IM (Intramuscular), all of the vaccines (1/5 dose of live vaccines) were injected into the chicken's thoracic muscle at 10 days post hatchery.

### II Experimental design

Specific Pathogen Free (SPF) fertilized eggs (n=60) were randomized into 12 groups. All groups (five eggs each group), all eggs were given 100 µl in volume each. 100 µg pCMV-NDF+ 100 µg pCMV-NDHN mixture was injected in each egg of group A, 100 µg pCMV-S1 was injected in each egg of group B, 100 µg pCMV-VP2 was injected in each egg of group C, 100 µg pCMV-NDF + 100 µg pCMV-NDHN+100 µg pCMV-S1(ND+IB) was injected in each egg of group D, 100 µg pCMV-NDF+ 100 µg pCMV-NDHN+100 µg pCMV-VP2 (ND+IBD) was injected in each egg of group E, 100µg pCMV-VP2+100 µg pCMV-VP2 mixture (IB+IBD) was injected in each egg of group F, 100 µg pCMV-NDF+ 100 µg pCMV-NDHN+100 µg pCMV-S1+100 µg pCMV-VP2 mixture (ND+IB+IBD) was injected in each egg of group G, one dose of commercialized in-ovo IBD vaccine (Embrex, Inc) was injected in each egg of group H as positive control, 100 ul PBS was injected in each egg of group I, J, K and L. All chickens in this experiment were given 100 µl in volume (1/5 dose of live vaccines), injected into the chicken's thoracic muscle each at 10 days post hatchery. Chickens in group A and I were injected with NDV vaccine, group B and J were injected with IBV vaccine, group C and K were injected with IBDV vaccine, group D were injected with the mixture of NDV+IB vaccines, group E were injected with the mixture ofNDV+IBD vaccines, group F were injected with the mixture of IB+IBD vaccines and group G and L were injected with the mixture of NDV, IB and IBD vaccines.

### III. Serology detection

All of the serum samples were collected at 10 days (injected with low dose live I vaccines at the same time), 17days, 24 days and 31 days post hatchery. The antibody titers were detected by ELISA using IB, IBD and NDV antibody test kits which purchased from IDEXX Laboratories, Inc. All of the samples were detected duplicated. Dilute test samples five hundred fold (1:500) with sample diluents prior to being assayed. The test procedure was applied according to the kit's manual. For the assay to be valid, measure and record absorbance values at 650 nm, A (650). The relative level of antibody in the unknown was determined by calculating the sample to positive (S/P) ratio. Endpoint titers were calculated using the formula: Log₁₀Titer = 1.09(Log₁₀S/P)+ 3.36

### Results

As shown in Table 1, the results demonstrated that, for the detection of anti-IBD antibodies, the IBDV recombinant antigens VP2 could be expressed and played the role of primary stimulation. The titers increased rapidly after a low dose vaccine booster. The titers of group C, E, F and G at 17 days post hatchery (i.e. 7 days post IM injection) were significantly higher than those of group K and L. Most importantly, the expression of IBDV antigen was not interfered by other monovalent DNA vaccines (NDV and IBV). The same results were also applied to IB and NDV DNA vaccines. The titers of group B, D, F and G were higher than those of group J and L at 17 days post hatchery (Table 2) and the titers of group A, D, E and G were higher than those of group I and L at 17 days post hatchery (Table 3). The only unpredicted result was the anti-NDV titer could not be highly induced by the triple valent DNA vaccine (Table 3, group G), but anti-IBD and anti-IB did (Tables 1 and 2, group G).

**Table 1. Serum Samples Detected by IDEXX IBD Antibody Test Kit (Ab Titers Correspond to the Average Titers ±SD)**

| Immunization and Sample Collection Schedule (days) | | | | |
|---|---|---|---|---|
| Animal Group | 10 Days PH* | 17 Days PH | 24 Days PH | 31 Days PH |
| C(IBD) | □** | 4535±1267 | 16623±3105 | 21254±3852 |
| E(IBD+ND) | □ | 1685±655 | 17339±2185 | 19041±2967 |
| F(IBD+IB) | □ | 8252±2205 | 10057±1295 | 17561±2006 |
| G(IBD+IB+ND) | □ | 9111±1701 | 13127±1763 | 16694±2134 |
| H(IBD positive) | 6553±851 | 13025±2131 | 18015±1592 | 18853±2614 |
| K(PBS/IBD) | □ | □ | 1853±302 | 17002±2965 |
| L(PBS/IBD+IB+ ND) | □ | □ | 6923±1168 | 18063±2531 |

| | | | | |
|---|---|---|---|---|
| *P H: post hatchery **□: average titers less than 396 (be considered negative by IDEXX kit) | | | | |

**Table 2. Serum Samples Detected by IDEXX IB Antibody test kit (Ab Titers Correspond to the Average Titers ±SD**

| Immunization and Sample Collection Schedule (days) | | | | |
|---|---|---|---|---|
| Animal Group | 10 Days PH* | 17 Days PH | 24 Days PH | 31 Days PH |
| B(IB) | □** | 441±117 | 2426±264 | 3214±877 |
| D(IB+ND) | □ | 586±182 | 805±221 | 1988±501 |
| F(IB+IBD) | □ | 509±89 | 685±186 | 1192±237 |
| G(IBD+IB+ND) | □ | 499±81 | 688±78 | 2551±531 |
| J(PBS/IB) | □ | □ | 485±76 | 1662±441 |
| L(PBS/IBD+IB+ ND) | □ | □ | 819±202 | 1332±488 |

| | | | | |
|---|---|---|---|---|
| *P H: post hatchery **□: average titers less than 396 (be considered negative by IDEXX kit) | | | | |

**Table 3. Serum Samples Detected by IDEXX ND Antibody Test Kit (Ab Titers Correspond to the Average Titers ±SD).**

| Immunization and Sample Collection Schedule (days) | | | | |
|---|---|---|---|---|
| Animal Group | 10 Days PH* | 17 Days PH | 24 Days PH | 31 Days PH |
| A(ND) | □ | 466±101 | 2394±456 | 8103±2198 |
| D(ND+IB) | □ | 706±140 | 1778±378 | 6811±2206 |
| E(ND+IBD) | □ | 517±104 | 3021±411 | 5991±1695 |
| G(IBD+IB+ND) | □ | □ | □ | 783±201 |
| I(PBS/ND) | □ | □ | 1853±324 | 3912±304 |
| L(PBS/IBD+IB+ ND) | □ | □ | 4027±662 | 5807±1996 |

| | | | | |
|---|---|---|---|---|
| *P H: post hatchery **□: average titers less than 396 (be considered negative by IDEXX kit) | | | | |

## Claims

1. A multiple DNA vaccine for *in ovo* injection comprising:
two or more DNA constructs, each DNA construct expressing an antigenic protein of an avian virus causing avian viral disease in fowl; wherein each of said DNA constructs comprises a DNA molecule encoding said antigenic protein of said avian virus and a vector which is a plasmid or a nucleic acid sequence, wherein the nucleic acid sequence is selected from the group consisting of nucleic acid sequences present in bacteriophages, cosmids and viruses; and wherein said DNA molecule is inserted into said vector.

2. The multiple DNA vaccine according to claim 1, wherein said avian virus is one selected from the group consisting of Marek's disease virus (MDV), infectious bursal disease virus (IBDV), Newcastle disease virus (NDV), infectious bronchitis virus (IBV), infectious laryngotracheitis virus (ILTV), avian encephalomyelitis (AEV), avian leukosis virus (ALV), fowlpox virus (FPV), avian parainfluenza virus (APV), duck hepatitis virus (DHV), and hemorrhagic enteritis virus (HEV).

3. The multiple DNA vaccine according to claim 1, wherein said plasmid is one selected from the group consistingof pcDNA3, pVAX1, pSectag, pTracer™, pDisplay™, pUC system plasmid, and pGEM^{®} system plasmid.

4. The multiple DNA vaccine according to claim 3, wherein said plasmid comprises a promoter which is selected from the group consisting of a CMV promoter, SV40 promoter, RSV promoter, and β-actin promoter.

5. The multiple DNA vaccine according to claim 1, wherein said DNA molecule is an entire gB gene of Marek's Disease virus (MDV) having the DNA sequence of SEQ ID NO: 1 or a fragment thereof.

6. The multiple DNA vaccine according to claim 1, wherein said DNA molecule is an entire VP2 gene of infectious bursal disease virus (IBDV) having the DNA sequence of SEQ ID NO: 2 or a fragment thereof.

7. The multiple DNA vaccine according to claim 1, wherein said DNA molecule is an entire HN gene of Newcastle disease virus (NDV) having the DNA sequence of bases 6321 to 8319 in SEQ ID NO: 3 or a fragment thereof.

8. The multiple DNA vaccine according to claim 1, wherein said DNA molecule is an entire S1 gene of infectious bronchitis virus (IBV) having the DNA sequence of SEQ ID NO: 4 or a fragment thereof.

9. The multiple DNA vaccine according to claim 1, wherein said DNA molecule is an entire glycoprotein G gene of infectious laryngotracheitis virus (ILTV) having the DNA sequence of SEQ ID NO: 5 or a fragment thereof.

10. The multiple DNA vaccine according to claim 1, wherein said DNA molecule is an entire VP1, or VP0, or VP3 gene of avian encephalomyelitis virus (AEV) or a fragment therof; wherein said VP1 gene has the DNA sequence of SEQ ID NO: 6; said VP0 gene has the DNA sequence of SEQ ID NO: 7; and said VP3 gene has the DNA sequence of SEQ ID NO: 8.

11. The multiple DNA vaccine according to claim 1, wherein said DNA molecule is an entire paraglycoprotein G gene of avian parainfluenza virus (APV) having the DNA sequence of SEQ ID NO: 9 or a fragment thereof.

12. The multiple DNA vaccine according to claim 1, wherein said DNA molecule is an entire type A penton base gene of hemorrhagic enteritis virus (HEV) having the DNA sequence of SEQ ID NO: 10 or a fragment thereof.

13. The multiple DNA vaccine according to claim 1, wherein said DNA molecule is an entire envelope antigen gene of fowlpox virus (FPV) having the DNA sequence of SEQ ID NO: 11 or a fragment thereof.

14. The multiple DNA vaccine according to claim 1, wherein said DNA vaccine comprises two DNA constructs, or three or more DNA constructs, each containing a DNA molecule capable of expressing a gene or a fragment thereof which is selected from the group consisting of gB gene of Marek's Disease virus (MDV) having the DNA sequence of SEQ ID NO: 1, VP2 gene of infectious bursal disease virus (IBDV) having the DNA sequence of SEQ ID NO: 2, HN gene of Newcastle disease virus (NDV) having the DNA sequence of bases 6321 to 8319 in SEQ ID NO: 3, S1 gene of infectious bronchitis virus (IBV) having the DNA sequence of SEQ ID NO: 4, glycoprotein G gene of infectious laryngotracheitis virus (ILTV) having the DNA sequence of SEQ ID NO: 5, VP1, or VP0, or VP3 gene of avian encephalomyelitis virus (AEV); wherein said VP1 gene has the DNA sequence of SEQ ID NO: 6; said VP0 gene has the DNA sequence of SEQ ID NO: 7; and said VP3 gene has the DNA sequence of SEQ ID NO: 8, envelope antigen gene of fowlpox virus (FPV) having the DNA sequence of SEQ ID NO: 11, paraglycoprotein G gene of avian parainfluenza virus (APV) having the DNA sequence of SEQ ID NO: 9, and type A penton base gene of hemorrhagic enteritis virus (HEV) having the DNA sequence of SEQ ID NO: 10.

15. The multiple DNA vaccine according to claim 1, wherein said fowl is one selected from the group consisting of chicken, turkey, duck, and goose.

16. A multiple DNA vaccine according to claim 1 for use in vaccinating fowl egg.

17. A method for preparing said multiple DNA vaccine according to claim 1 comprising:
ligating a DNA molecule to a vector according to claim 1 to form a DNA construct;
mixing two or more said DNA constructs to form said multiple DNA vaccine;
wherein said DNA molecule comprises a gene or a fragment thereof which is selected from the group consisting of gB gene of Marek's Disease virus (MDV) having the DNA sequence of SEQ ID NO: 1, VP2 gene of infectious bursal disease virus (IBDV) having the DNA sequence of SEQ ID NO: 2, HN gene of Newcastle disease virus (NDV) having the DNA sequence of bases 6321 to 8319 in SEQ ID NO: 3, S1 gene of infectious bronchitis virus (IBV) having the DNA sequence of SEQ ID NO: 4, glycoprotein G gene of infectious laryngotracheitis virus (ILTV) having the DNA sequence of SEQ ID NO: 5, VP1, or VP0, or VP3 gene of avian encephalomyelitis virus (AEV); wherein said VP1 gene has the DNA sequence of SEQ ID NO: 6; said VP0 gene has the DNA sequence of SEQ ID NO: 7; and said VP3 gene has the DNA sequence of SEQ ID NO: 8, envelope antigen gene of fowlpox virus (FPV) having the DNA sequence of SEQ ID NO: 11, paraglycoprotein G gene of avian parainfluenza virus (APV) having the DNA sequence of SEQ ID NO: 9, and type A penton base gene of hemorrhagic enteritis virus (HEV) having the DNA sequence of SEQ ID NO: 10.

18. A multivalent DNA vaccine for *in ovo* injection comprising:
a DNA construct containing two or more DNA molecules, wherein each of said DNA molecules express an antigenic protein of an avian virus causing avian viral disease in fowl; wherein each of said DNA constructs comprises a DNA molecule encoding said antigenic protein of said avian virus and a vector which is a plasmid or a nucleic acid sequence, wherein the nucleic acid sequence is selected from the group consisting of nucleic acid sequences present in bacteriophages, cosmids and viruses; and wherein said DNA molecule is inserted into said vector.

19. The multivalent DNA vaccine according to claim 18, wherein said DNA molecules is a gene or a fragment thereof which is selected from the group consisting of gB gene of Marek's Disease virus (MDV) having the DNA sequence of SEQ ID NO: 1, VP2 gene of infectious bursal disease virus (IBDV) having the DNA sequence of SEQ ID NO: 2, HN gene of Newcastle disease virus (NDV) having the DNA sequence of bases 6321 to 8319 in SEQ ID NO: 3, S1 gene of infectious bronchitis virus (IBV) having the DNA sequence of SEQ ID NO: 4, glycoprotein G gene of infectious laryngotracheitis virus (ILTV) having the DNA sequence of SEQ ID NO: 5, VP1, or VP0, or VP3 gene of avian encephalomyelitis virus (AEV); wherein said VP1 gene has the DNA sequence of SEQ ID NO: 6; said VP0 gene has the DNA sequence of SEQ ID NO: 7; and said VP3 gene has the DNA sequence of SEQ ID NO: 8, envelope antigen gene of fowlpox virus (FPV) having the DNA sequence of SEQ ID NO: 11, paraglycoprotein G gene of avian parainfluenza virus (APV) having the DNA sequence of SEQ ID NO: 9, and type A penton base gene of hemorrhagic enteritis virus (HEV) having the DNA sequence of SEQ ID NO: 10.

20. The multivalent DNA vaccine according to claim 18, wherein said plasmid is one selected from the group consisting of pcDNA3, pVAX1, pSectag, pTracer™, pDisplay™, pUC system plasmid, and pGEM^{®} system plasmid.

21. The multiple DNA vaccine according to claim 18, wherein said plasmid comprises a promoter which is selected from the group consisting of a CMV promoter, SV40 promoter, RSV promoter, and β-actin promoter.

22. A multivalent DNA vaccine according to claim 18 for use in vaccinating fowl egg.

## Patentansprüche

1. Ein Mehrfach-DNA-Vakzin für *die in ovo* Injektion, umfassend:
zwei oder mehr DNA-Konstrukte, wobei jedes DNA-Konstrukt ein antigenes Protein eines aviären Virus, das die Vogelgrippe Viruserkrankung in Geflügel verursacht, exprimiert; wobei jedes der DNA-Konstrukte ein DNA-Molekül umfasst, das das antigene Protein des aviären Virus und einen Vektor, der ein Plasmid oder eine Nukleinsäuresequenz ist, kodiert, wobei die Nukleinsäuresequenz aus der Gruppe bestehend aus Nukleinsäuresequenzen, die in Bakteriophagen, Cosmiden und Viren vorhanden sind ausgewählt ist; und wobei das DNA-Molekül in den Vektor insertiert ist.

2. Das Mehrfach-DNA-Vakzin nach Anspruch 1, wobei das aviäre Virus aus der Gruppe bestehend aus Marek-Krankheit-Virus (MDV), Virus der infektiösen Bursitis (IBDV), Newcastle Krankheit Virus (NDV), Virus der infektiösen Bronchitis (IBV), Virus der infektiösen Laryngotracheitis (ILTV), Aviäre Enzephalomyelitis Virus (AEV), Aviäres Leukose-Virus (ALV), Geflügelpockenvirus (FPV), Vogel Parainfluenzavirus (APV), Ente Hepatitis-Virus (DHV) und hämorrhagisches Enteritis-Virus (HEV) ausgewählt wird.

3. Das Mehrfach-DNA-Vakzin nach Anspruch 1, wobei das Plasmid eines ist, das aus der Gruppe bestehend aus pcDNA3, pVAX1, pSectag, pTracer™, pDisplay™, dem pUC-Plasmid-System und dem pGEM^{®}-Plasmid-System ausgewählt ist.

4. Das Mehrfach-DNA-Vakzin nach Anspruch 3, wobei das Plasmid einen Promotor umfasst, der aus der Gruppe bestehend aus einem CMV-Promotor, SV40-Promotor, RSV-Promotor und β-Actin-Promotor ausgewählt wird.

5. Das Mehrfach-DNA-Vakzin nach Anspruch 1, wobei das DNA-Molekül ein ganzes gB-Gen des Marek-Krankheit-Viruses (MDV), das die DNA-Sequenz von SEQ ID NO: 1 oder einem Fragment davon hat, wird.

6. Das Mehrfach-DNA-Vakzin nach Anspruch 1, wobei das DNA-Molekül ein ganzes VP2-Gen des Viruses der infektiösen Bursitis (IBDV), das die DNA-Sequenz von SEQ ID NO: 2 oder einem Fragment davon hat, wird.

7. Das Mehrfach-DNA-Vakzin nach Anspruch 1, wobei das DNA-Molekül ein ganzes HN-Gen des Newcastle Krankheit Viruses (NDV), das die DNA-Sequenz der Basen 6321 bis 8319 aus SEQ ID NO: 3 oder einem Fragment davon hat, ist.

8. Das Mehrfach-DNA-Vakzin nach Anspruch 1, wobei das DNA-Molekül ein ganzes S1 Gen des Viruses der infektiösen Bronchitis (IBV), das die DNA-Sequenz von SEQ ID NO: 4 oder einem Fragment davon hat, ist.

9. Das Mehrfach-DNA-Vakzin nach Anspruch 1, wobei das DNA-Molekül ein ganzes Glycoprotein G-Gen des infektiösen Laryngotracheitis-Virus (ILTV), das die DNA-Sequenz von SEQ ID NO: 5 oder einem Fragment davon hat, ist.

10. Das Mehrfach-DNA-Vakzin nach Anspruch 1, wobei das DNA-Molekül ein ganzes VP1, VP0 oder VP3-Gen des Vogel-Encephalomyelitis-Viruses (AEV) oder ein Fragment davon ist, wobei das VP1-Gen die DNA-Sequenz von SEQ ID NO: 6; das VP0-Gen die DNA-Sequenz von SEQ ID NO: 7, und das VP3-Gen die DNA-Sequenz von SEQ ID NO: 8 hat.

11. Das Mehrfach-DNA-Vakzin nach Anspruch 1, wobei das DNA-Molekül ein ganzes Paraglycoprotein G-Gen des Vogel-Parainfluenza-Virus (APV), das die DNA-Sequenz von SEQ ID NO: 9 oder einem Fragment davon hat, ist.

12. Das Mehrfach-DNA-Vakzin nach Anspruch 1, wobei das DNA-Molekül ein ganzes Typ A Pentonbasen Gen des hämorrhagischen Enteritis-Virus (HEV), das die DNA-Sequenz von SEQ ID NO: 10 oder einem Fragment davon hat, ist.

13. Das Mehrfach-DNA-Vakzin nach Anspruch 1, wobei das DNA-Molekül ein ganzes Hüllantigen-Gen des Geflügelpockenviruses (FPV), das die DNA-Sequenz von SEQ ID NO: 11 oder einem Fragment davon hat, ist.

14. Das Mehrfach-DNA-Vakzin nach Anspruch 1, wobei das DNA-Vakzin zwei DNA-Konstrukte oder drei oder mehr DNA-Konstrukte umfasst, wobei jedes ein DNA-Molekül enthält, das in der Lage ist ein Gen oder ein Fragment davon zu exprimieren, das aus der Gruppe bestehend aus dem gB-Gen des Marek-Krankheit-Viruses (MDV), das die DNA-Sequenz von SEQ ID NO: 1 oder einem Fragment davon hat, dem P2-Gen des Viruses der infektiösen Bursitis (IBDV), das die DNA-Sequenz von SEQ ID NO: 2 oder einem Fragment davon hat, dem HN-Gen des Newcastle Krankheit Viruses (NDV), das die DNA-Sequenz der Basen 6321 bis 8319 aus SEQ ID NO: 3 oder einem Fragment davon hat, dem S1 Gen des Viruses der infektiösen Bronchitis (IBV), das die DNA-Sequenz von SEQ ID NO: 4 oder einem Fragment davon hat, dem Glycoprotein G-Gen des infektiösen Laryngotracheitis-Virus (ILTV), das die DNA-Sequenz von SEQ ID NO: 5 oder einem Fragment davon hat, dem VP1, VP0 oder VP3-Gen des Vogel-Encephalomyelitis-Viruses (AEV), wobei das VP1-Gen die DNA-Sequenz von SEQ ID NO: 6 hat; das VP0-Gen die DNA-Sequenz von SEQ ID NO: 7 hat; und das VP3-Gen die DNA-Sequenz von SEQ ID NO: 8 hat, dem Hüllantigen-Gen des Geflügelpockenviruses (FPV), das die DNA-Sequenz von SEQ ID NO: 11 oder einem Fragment davon hat, dem Paraglycoprotein G-Gen des Vogel-Parainfluenza-Virus (APV), das die DNA-Sequenz von SEQ ID NO: 9 oder einem Fragment davon hat, und dem Typ A Pentonbasen Gen des hämorrhagischen Enteritis-Virus (HEV), das die DNA-Sequenz von SEQ ID NO: 10 oder einem Fragment davon hat, ausgewählt wird.

15. Das Mehrfach-DNA-Vakzin nach Anspruch 1, wobei das Geflügel aus der Gruppe bestehend aus Huhn, Truthahn, Ente und Gans ausgewählt wird.

16. Das Mehrfach-DNA-Vakzin nach Anspruch 1 zur Verwendung in der Vakzination eines Geflügeleis.

17. Ein Verfahren zur Herstellung des Mehrfach-DNA-Vakzins nach Anspruch 1 umfassend:
Ligieren eines DNA-Moleküls an einen Vektor nach Anspruch 1 um ein DNA-Konstrukt zu bilden; Vermischen von zwei oder mehr der DNA-Konstrukte um das Mehrfach-DNA-Vakzin zu bilden; wobei das DNA-Molekül ein Gen oder ein Fragment davon umfasst, das aus der Gruppe bestehend aus dem gB-Gen des Marek-Krankheit-Viruses (MDV), das die DNA-Sequenz von SEQ ID NO: 1 oder einem Fragment davon hat, dem P2-Gen des Viruses der infektiösen Bursitis (IBDV), das die DNA-Sequenz von SEQ ID NO: 2 oder einem Fragment davon hat, dem HN-Gen des Newcastle Krankheit Viruses (NDV), das die DNA-Sequenz der Basen 6321 bis 8319 aus SEQ ID NO: 3 oder einem Fragment davon hat, dem S1 Gen des Viruses der infektiösen Bronchitis (IBV), das die DNA-Sequenz von SEQ ID NO: 4 oder einem Fragment davon hat, dem Glycoprotein G-Gen des infektiösen Laryngotracheitis-Virus (ILTV), das die DNA-Sequenz von SEQ ID NO: 5 oder einem Fragment davon hat, dem VP1, VP0 oder VP3-Gen des Vogel-Encephalomyelitis-Viruses (AEV), wobei das VP1-Gen die DNA-Sequenz von SEQ ID NO: 6 hat; das VP0-Gen die DNA-Sequenz von SEQ ID NO: 7 hat; und das VP3-Gen die DNA-Sequenz von SEQ ID NO: 8 hat, dem Hüllantigen-Gen des Geflügelpockenviruses (FPV), das die DNA-Sequenz von SEQ ID NO: 11 oder einem Fragment davon hat, dem Paraglycoprotein G-Gen des Vogel-Parainfluenza-Virus (APV), das die DNA-Sequenz von SEQ ID NO: 9 oder einem Fragment davon hat, und dem Typ A Pentonbasen Gen des hämorrhagischen Enteritis-Virus (HEV), das die DNA-Sequenz von SEQ ID NO: 10 oder einem Fragment davon hat, ausgewählt wird.

18. Ein multivalentes DNA-Vakzin für die *in ovo* Injektion, umfassend:
ein DNA-Konstrukt, das zwei oder mehr DNA-Moleküle enthält, wobei jedes DNA-Molekül ein antigenes Protein eines aviären Virus, das die Vogelgrippe Viruserkrankung in Geflügel verursacht, exprimiert; wobei jedes der DNA-Konstrukte ein DNA-Molekül umfasst, das das antigene Protein des aviären Virus und einen Vektor, der ein Plasmid oder eine Nukleinsäuresequenz ist, kodiert, wobei die Nukleinsäuresequenz aus der Gruppe bestehend aus Nukleinsäuren, die in Bakteriophagen, Cosmiden und Viren vorhanden sind ausgewählt ist; und wobei das DNA-Molekül in den Vektor insertiert ist.

19. Das multivalente DNA-Vakzin nach Anspruch 18, wobei das DNA-Molekül ein Gen oder ein Fragment davon umfasst, das aus der Gruppe bestehend aus dem gB-Gen des Marek-Krankheit-Viruses (MDV), das die DNA-Sequenz von SEQ ID NO: 1 oder einem Fragment davon hat, dem P2-Gen des Viruses der infektiösen Bursitis (IBDV), das die DNA-Sequenz von SEQ ID NO: 2 oder einem Fragment davon hat, dem HN-Gen des Newcastle Krankheit Viruses (NDV), das die DNA-Sequenz der Basen 6321 bis 8319 aus SEQ ID NO: 3 oder einem Fragment davon hat, dem S1 Gen des Viruses der infektiösen Bronchitis (IBV), das die DNA-Sequenz von SEQ ID NO: 4 oder einem Fragment davon hat, dem Glycoprotein G-Gen des infektiösen Laryngotracheitis-Virus (ILTV), das die DNA-Sequenz von SEQ ID NO: 5 oder einem Fragment davon hat, dem VP1, VP0 oder VP3-Gen des Vogel-Encephalomyelitis-Viruses (AEV), wobei das VP1-Gen die DNA-Sequenz von SEQ ID NO: 6 hat; das VP0-Gen die DNA-Sequenz von SEQ ID NO: 7 hat; und das VP3-Gen die DNA-Sequenz von SEQ ID NO: 8 hat, dem Hüllantigen-Gen des Geflügelpockenviruses (FPV), das die DNA-Sequenz von SEQ ID NO: 11 oder einem Fragment davon hat, dem Paraglycoprotein G-Gen des Vogel-Parainfluenza-Virus (APV), das die DNA-Sequenz von SEQ ID NO: 9 oder einem Fragment davon hat, und dem Typ A Pentonbasen Gen des hämorrhagischen Enteritis-Virus (HEV), das die DNA-Sequenz von SEQ ID NO: 10 oder einem Fragment davon hat, ausgewählt ist.

20. Das multivalente DNA-Vakzin nach Anspruch 18, wobei das Plasmid eines ist, das aus der Gruppe bestehend aus pcDNA3, pVAX1, pSectag, pTracer™, pDisplay™, dem pUC-Plasmid-System und dem pGEM^{®}-Plasmid-System ausgewählt wird.

21. Das multivalente DNA-Vakzin nach Anspruch 18, wobei das Plasmid einen Promotor umfasst, der aus der Gruppe bestehend aus einem CMV-Promotor, SV40-Promotor, RSV-Promotor und β-Actin-Promotor ausgewählt wird.

22. Das multivalente DNA-Vakzin nach Anspruch 18 zur Verwendung in der Vakzination eines Geflügeleis.

## Revendications

1. Vaccin à ADN multiple pour *injection in ovo* comprenant :
deux constructions d'ADN ou plus, chaque construction d'ADN exprimant une protéine antigénique d'un virus aviaire causant une maladie virale aviaire chez des volailles ; chacune desdites constructions d'ADN comprenant une molécule d'ADN codant pour ladite protéine antigénique dudit virus aviaire et un vecteur qui est un plasmide ou une séquence d'acide nucléique, la séquence d'acide nucléique étant choisie dans le groupe constitué de séquences d'acide nucléique présentes dans des bactériophages, des cosmides et des virus ; et ladite molécule d'ADN étant insérée dans ledit vecteur.

2. Vaccin à ADN multiple selon la revendication 1, ledit virus aviaire étant l'un choisi dans le groupe constitué du virus de la maladie de Marek (MDV), le virus de la bursite infectieuse (IBDV), le virus de la maladie de Newcastle (NDV), le virus de la bronchite infectieuse (IBV), le virus de la laryngotrachéite infectieuse (ILTV), l'encéphalomyélite aviaire (AEV), le virus de la leucose aviaire (ALV), le virus de la variole aviaire (FPV), le virus parainfluenza aviaire (APV), le virus de l'hépatite du canard (DHV), et le virus de l'entérite hémorragique (HEV).

3. Vaccin à ADN multiple selon la revendication 1, ledit plasmide étant l'un choisi dans le groupe constitué de pcDNA3, de pVAX1, de pSectag, de pTracer™, de pDisplay™, du système de plasmide pUC, et du système de plasmide pGEM^{®}.

4. Vaccin à ADN multiple selon la revendication 3, ledit plasmide comprenant un promoteur qui est choisi dans le groupe constitué d'un promoteur de CMV, un promoteur de SV40, un promoteur de RSV, et un promoteur de β-actine.

5. Vaccin à ADN multiple selon la revendication 1, ladite molécule d'ADN étant un gène gB entier du virus de la maladie de Marek (MDV) ayant la séquence d'ADN de SEQ ID NO: 1 ou un fragment de celui-ci.

6. Vaccin à ADN multiple selon la revendication 1, ladite molécule d'ADN étant un gène VP2 entier du virus de la bursite infectieuse (IBDV) ayant la séquence d'ADN de SEQ ID NO: 2 ou un fragment de celui-ci.

7. Vaccin à ADN multiple selon la revendication 1, ladite molécule d'ADN étant un gène HN entier du virus de la maladie de Newcastle (NDV) ayant la séquence d'ADN des bases 6321 à 8319 dans SEQ ID NO: 3 ou un fragment de celui-ci.

8. Vaccin à ADN multiple selon la revendication 1, ladite molécule d'ADN étant un gène S1 entier du virus de la bronchite infectieuse (IBV) ayant la séquence d'ADN de SEQ ID NO: 4 ou un fragment de celui-ci.

9. Vaccin à ADN multiple selon la revendication 1, ladite molécule d'ADN étant un gène de glycoprotéine G entier du virus de la laryngotrachéite infectieuse (ILTV) ayant la séquence d'ADN de SEQ ID NO: 5 ou un fragment de celui-ci.

10. Vaccin à ADN multiple selon la revendication 1, ladite molécule d'ADN étant un gène VP1, ou VP0, ou VP3 entier du virus de l'encéphalomyélite aviaire (AEV) ou un fragment de celui-ci ; ledit gène VP1 ayant la séquence d'ADN de SEQ ID NO: 6 ; ledit gène VP0 ayant la séquence d'ADN de SEQ ID NO: 7 ; et ledit gène VP3 ayant la séquence d'ADN de SEQ ID NO: 8.

11. Vaccin à ADN multiple selon la revendication 1, ladite molécule d'ADN étant un gène de paraglycoprotéine G entier du virus parainfluenza aviaire (APV) ayant la séquence d'ADN de SEQ ID NO: 9 ou un fragment de celui-ci.

12. Vaccin à ADN multiple selon la revendication 1, ladite molécule d'ADN étant un gène de base du penton de type A entier du virus de l'entérite hémorragique (HEV) ayant la séquence d'ADN de SEQ ID NO: 10 ou un fragment de celui-ci.

13. Vaccin à ADN multiple selon la revendication 1, ladite molécule d'ADN étant un gène d'antigène d'enveloppe entier du virus de la variole aviaire (FPV) ayant la séquence d'ADN de SEQ ID NO: 11 ou un fragment de celui-ci.

14. Vaccin à ADN multiple selon la revendication 1, ledit vaccin à ADN comprenant deux constructions d'ADN, ou trois constructions d'ADN ou plus, chacune contenant une molécule d'ADN capable d'exprimer un gène ou un fragment de celui-ci qui est choisi dans le groupe constitué du gène gB du virus de la maladie de Marek (MDV) ayant la séquence d'ADN de SEQ ID NO: 1, du gène VP2 du virus de la bursite infectieuse (IBDV) ayant la séquence d'ADN de SEQ ID NO: 2, du gène HN gène du virus de la maladie de Newcastle (NDV) ayant la séquence d'ADN des bases 6321 à 8319 dans SEQ ID NO: 3, du gène S1 du virus de la bronchite infectieuse (IBV) ayant la séquence d'ADN de SEQ ID NO:4, du gène de glycoprotéine G du virus de la laryngotrachéite infectieuse (ILTV) ayant la séquence d'ADN de SEQ ID NO:5, du gène VP1, ou VP0, ou VP3 du virus de l'encéphalomyélite aviaire (AEV) ; ledit gène VP1 ayant la séquence d'ADN de SEQ ID NO: 6 ; ledit gène VP0 ayant la séquence d'ADN de SEQ ID NO: 7 ; et ledit gène VP3 ayant la séquence d'ADN de SEQ ID NO: 8, du gène d'antigène d'enveloppe du virus de la variole aviaire (FPV) ayant la séquence d'ADN de SEQ ID NO: 11, du gène de paraglycoprotéine G du virus parainfluenza aviaire (APV) ayant la séquence d'ADN de SEQ ID NO: 9, et du gène de base du penton de type A du virus de l'entérite hémorragique (HEV) ayant la séquence d'ADN de SEQ ID NO: 10.

15. Vaccin à ADN multiple selon la revendication 1, ladite volaille étant l'une choisie dans le groupe constitué d'un poulet, une dinde, un canard et une oie.

16. Vaccin à ADN multiple selon la revendication 1 pour utilisation dans la vaccination d'oeufs de volaille.

17. Procédé pour préparer ledit vaccin à ADN multiple selon la revendication 1 comprenant :
la ligature d'une molécule d'ADN à un vecteur selon la revendication 1 pour former une construction d'ADN ;
le mélange de deux ou plus desdites constructions d'ADN pour former ledit vaccin à ADN multiple ;
ladite molécule d'ADN comprenant un gène ou un fragment de celui-ci qui est choisi dans le groupe constitué du gène gB du virus de la maladie de Marek (MDV) ayant la séquence d'ADN de SEQ ID NO: 1, du gène VP2 du virus de la bursite infectieuse (IBDV) ayant la séquence d'ADN de SEQ ID NO: 2, du gène HN du virus de la maladie de Newcastle (NDV) ayant la séquence d'ADN des bases 6321 à 8319 dans SEQ ID NO: 3, du gène S1 du virus de la bronchite infectieuse (IBV) ayant la séquence d'ADN de SEQ ID NO: 4, du gène de glycoprotéine G du virus de la laryngotrachéite infectieuse (ILTV) ayant la séquence d'ADN de SEQ ID NO:5, du gène VP1, ou VP0, ou VP3 du virus de l'encéphalomyélite aviaire (AEV) ; ledit gène VP1 ayant la séquence d'ADN de SEQ ID NO: 6 ; ledit gène VP0 ayant la séquence d'ADN de SEQ ID NO: 7 ; et ledit gène VP3 ayant la séquence d'ADN de SEQ ID NO: 8, du gène d'antigène d'enveloppe du virus de la variole aviaire (FPV) ayant la séquence d'ADN de SEQ ID NO: 11, du gène de paraglycoprotéine G du virus parainfluenza aviaire (APV) ayant la séquence d'ADN de SEQ ID NO: 9, et du gène de base du penton de type A du virus de l'entérite hémorragique (HEV) ayant la séquence d'ADN de SEQ ID NO: 10.

18. Vaccin à ADN multivalent pour injection *in ovo* comprenant :
une construction d'ADN contenant deux molécules d'ADN ou plus, chacune desdites molécules d'ADN exprimant une protéine antigénique d'un virus aviaire causant une maladie virale aviaire chez des volailles ; chacune desdites constructions d'ADN comprenant une molécule d'ADN codant pour ladite protéine antigénique dudit virus aviaire et un vecteur qui est un plasmide ou une séquence d'acide nucléique, la séquence d'acide nucléique étant choisie dans le groupe constitué de séquences d'acide nucléique présentes dans des bactériophages, des cosmides et des virus ; et ladite molécule d'ADN étant insérée dans ledit vecteur.

19. Vaccin à ADN multivalent selon la revendication 18, lesdites molécules d'ADN étant un gène ou un fragment de celui-ci qui est choisi dans le groupe constitué du gène gB du virus de la maladie de Marek (MDV) ayant la séquence d'ADN de SEQ ID NO: 1, du gène VP2 du virus de la bursite infectieuse (IBDV) ayant la séquence d'ADN de SEQ ID NO: 2, du gène HN du virus de la maladie de Newcastle (NDV) ayant la séquence d'ADN des bases 6321 à 8319 dans SEQ ID NO: 3, du gène S1 du virus de la bronchite infectieuse (IBV) ayant la séquence d'ADN de SEQ ID NO: 4, du gène de glycoprotéine G du virus de la laryngotrachéite infectieuse (ILTV) ayant la séquence d'ADN de SEQ ID NO: 5, du gène VP1, ou VP0, ou VP3 du virus de l'encéphalomyélite aviaire (AEV) ; ledit gène VP1 ayant la séquence d'ADN de SEQ ID NO: 6 ; ledit gène VP0 ayant la séquence d'ADN de SEQ ID NO: 7 ; et ledit gène VP3 ayant la séquence d'ADN de SEQ ID NO: 8, du gène d'antigène d'enveloppe du virus de la variole aviaire (FPV) ayant la séquence d'ADN de SEQ ID NO: 11, du gène de paraglycoprotéine G du virus parainfluenza aviaire (APV) ayant la séquence d'ADN de SEQ ID NO: 9, et du gène de base du penton de type A du virus de l'entérite hémorragique (HEV) ayant la séquence d'ADN de SEQ ID NO: 10.

20. Vaccin à ADN multivalent selon la revendication 18, ledit plasmide étant l'un choisi dans le groupe constitué de pcDNA3, de pVAX1, de pSectag, de pTracer™, de pDisplay™, du système de plasmide pUC, et du système de plasmide pGEM^{®}.

21. Vaccin à ADN multiple selon la revendication 18, ledit plasmide comprenant un promoteur qui est choisi dans le groupe constitué d'un promoteur de CMV, un promoteur de SV40, un promoteur de RSV, et un promoteur de β-actine.

22. A multivalent vaccin à ADN selon la revendication 18 pour utilisation dans la vaccination d'oeufs de volaille.
